(19) Europäisches Patentamt
European Patent Office
Office européen des brevets

(11) EP 3 810 076 B1

(12) FASCICULE DE BREVET EUROPEEN

(45) Date de publication et mention de la délivrance du brevet:
**03.08.2022 Bulletin 2022/31**

(21) Numéro de dépôt: **19731741.5**

(22) Date de dépôt: **20.06.2019**

(51) Classification Internationale des Brevets (IPC):
***A61K 8/41*** *(2006.01)* ***A61Q 5/10*** *(2006.01)*
***A61K 8/73*** *(2006.01)*

(52) Classification Coopérative des Brevets (CPC):
**A61K 8/73; A61K 8/41; A61Q 5/10**

(86) Numéro de dépôt international:
**PCT/EP2019/066364**

(87) Numéro de publication internationale:
**WO 2019/243508 (26.12.2019 Gazette 2019/52)**

(54) **PROCEDE DE COLORATION CAPILLAIRE METTANT EN OEUVRE UNE COMPOSITION COLORANTE ET UNE COMPOSITION OXYDANTE, LESDITES COMPOSITIONS COMPRENANT UNE GOMME DE SCLEROGLUCANE**

HAARFÄRBEVERFAHREN UNTER VERWENDUNG EINER FÄRBEZUSAMMENSETZUNG UND EINER OXIDIERENDEN ZUSAMMENSETZUNG, WOBEI DIE ZUSAMMENSETZUNGEN EINEN SCLEROGLUCANGUMMI UMFASSEN

HAIR DYEING METHOD USING A DYE COMPOSITION AND AN OXIDIZING COMPOSITION, SAID COMPOSITIONS COMPRISING A SCLEROGLUCAN GUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2018 FR 1855429**

(43) Date de publication de la demande:
**28.04.2021 Bulletin 2021/17**

(73) Titulaire: **L'OREAL**
**75008 Paris (FR)**

(72) Inventeurs:
- **MULLER, Sabrina**
  **93400 SAINT-OUEN (FR)**
- **CHARRIER, Delphine**
  **93400 SAINT-OUEN (FR)**
- **PIZZINO, Aldo**
  **93400 SAINT-OUEN (FR)**
- **SIMONET, Frédéric**
  **93400 SAINT-OUEN (FR)**
- **MILIC, Mladen**
  **93400 SAINT-OUEN (FR)**
- **YADEL, Cindy**
  **93400 SANT-OUEN (FR)**
- **CARDONNEL, Fanny**
  **93400 SAINT-OUEN (FR)**

(74) Mandataire: **L'Oreal**
**Service D.I.P.I.**
**9, rue Pierre Dreyfus**
**92110 Clichy (FR)**

(56) Documents cités:
**US-A1- 2010 192 969**

- **DATABASE GNPD [Online] MINTEL; 11 mai 2011 (2011-05-11), anonymous: "Colourant Cream", XP055547325, extrait de www.gnpd.com Database accession no. 1533817**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 810 076 B1

## Description

La présente invention concerne un procédé de coloration des fibres kératiniques, en particulier des fibres kératiniques humaines telles que les cheveux mettant en œuvre l'appliquer sur les fibres d'une composition colorante (A) comprenant un ou plusieurs colorant(s) d'oxydation, une ou plusieurs gommes de scléroglucane en une teneur totale en poids supérieure ou égale à 0,5% par rapport au poids total de la composition (A) et un ou plusieurs agents alcalins, et d'une composition oxydante (B) comprenant un ou plusieurs agent(s) oxydant(s) chimique(s) et une ou plusieurs gommes de scléroglucane de préférence en une teneur totale en poids supérieure ou égale à 0,5% par rapport au poids total de la composition (B), la composition oxydante (B) étant mélangée avec la composition colorante(A) juste avant l'emploi (application sur lesdites fibres) (extemporanément).

L'invention concerne également un dispositif à plusieurs compartiments, approprié pour la mise en œuvre dudit procédé de coloration.

La présente invention a trait au domaine de la coloration des fibres kératiniques et plus particulièrement à celui de la coloration capillaire.

Parmi les méthodes de coloration des fibres kératiniques humaines, telles que les cheveux, on peut citer la coloration d'oxydation ou permanente. Plus particulièrement, ce mode de coloration met en œuvre un ou plusieurs colorants d'oxydation, habituellement une ou plusieurs bases d'oxydation éventuellement associées à un ou plusieurs coupleurs.

En général, des bases d'oxydation sont choisies parmi les ortho- ou para-phénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Il est également possible d'ajouter à ces compositions, des colorants directs, qui sont des molécules colorées et colorantes ayant une affinité pour les fibres. Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. La présence de tels composés permet d'enrichir encore la coloration obtenue, en reflets ou bien d'augmenter la chromaticité de la coloration obtenue.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

La coloration d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Le procédé de coloration doit également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine, afin d'obtenir une coloration la plus homogène possible des fibres kératiniques. Les compositions de coloration doivent également permettre de conférer de bonnes propriétés cosmétiques aux fibres kératiniques, en particulier du soin, de la douceur et/ou de la discipline, présenter de bonnes qualités d'usage, en particulier être faciles à appliquer, tout en permettant d'atteindre des résultats couleur visibles (c'est-à-dire notamment intenses, chromatiques), homogènes et tenaces.

Les compositions mises en œuvre dans un procédé de coloration doivent, en outre, présenter de bonnes propriétés de mélange et d'application sur les fibres kératiniques, et notamment de bonnes propriétés rhéologiques pour ne pas couler, lors de leur application, sur le visage, le cuir chevelu, ou en dehors des zones que l'on se propose de teindre, cela permet notamment une application homogène des racines jusqu'au pointes.

En particulier on cherche à obtenir des compositions de coloration ou oxydantes stables dans le temps pendant plusieurs semaines. Par « stable » au sens de la présente invention on entend en particulier que des caractéristiques physiques telles que l'aspect, le pH et/ou la viscosité varient peu, voire ne varient pas, au court du temps, en particulier, que la viscosité de la composition n'évolue pas ou peu pendant le stockage et/ou que la composition ne déphase pas pendant le stockage.

En effet, il est souhaitable que les compositions de coloration ou oxydantes soient stables dans le temps, en particulier stables après 1 mois à 45°C, voire après 2 mois à 45°C.

On cherche également à obtenir des compositions de coloration stables sur une large gamme de pH et en particulier vis-à-vis de pH extrêmes, par exemple à des pH alcalins allant de 9 à 12. Enfin, les compositions de coloration peuvent parfois être déstabilisées (déphasées) par des teneurs élevés en certains composés, et il est donc souhaitable que ces compositions soient stables dans ces conditions, en particulier qui ne déphase pas. US 2010/192969 A1 (DEGEORGE MICHAEL [US] ET AL) 5 août 2010 (2010-08-05) décrit un kit à plusieurs compartiments pour la coloration capillaire, comprenant au moins deux compartiments, dont l'un contenant une composition comprenant de la gomme de scléroglucane.

Ainsi l'un des objectifs de la présente invention est de proposer un procédé de coloration des fibres kératiniques, de préférence des fibres kératiniques humaines telles que les cheveux qui ne présentent pas les inconvénients mentionnés ci-dessus, c'est-à-dire qui est capable de conduire à de très bonnes performances de coloration, notamment en termes d'intensité et/ou de montée de la couleur, ainsi qu'en terme de sélectivité, de chromaticité et/ou de rémanence aux agents extérieurs, présentant de bonnes qualités d'usage en particulier lors de leur application sur les fibres kératiniques, et conférant de bonnes propriétés cosmétiques aux fibres (douceur, lissage) les compositions mises en œuvre étant stables (notamment ne déphasant pas et/ou dont la viscosité ou le pH n'évolue(nt) pas ou peu dans le temps).

De façon avantageuse, les compositions de coloration et/ou oxydantes mises en œuvre dans le procédé selon l'invention sont translucides. En effet, un produit de coloration translucide offre la possibilité au consommateur de visualiser l'évolution du résultat couleur pendant le temps de pause du produit lui laissant le choix de stopper au moment où le résultat lui convient. De plus, grâce à leurs propriétés rhéologiques très stables et proches des compositions colorantes et oxydantes mises en œuvre et permettent éventuellement l'utilisation d'un conditionnement en aérosol pour la mise en œuvre du procédé selon l'invention.

Ces buts et d'autres sont atteints par la présente invention qui a donc pour objet un procédé de coloration des fibres kératiniques, de préférence des fibres kératiniques humaines telles que les cheveux, mettant en œuvre l'application sur les fibres:

a) d'une composition colorante (A) comprenant :

- un ou plusieurs colorant(s) d'oxydation ;
- une ou plusieurs gommes de scléroglucane, en une teneur totale supérieure ou égale à 0,5% en poids, par rapport au poids de la composition ;
- un ou plusieurs agents alcalins ; et

b) d'une composition oxydante (B) comprenant :

- un ou plusieurs agent(s) oxydants chimiques, de préférence choisi(s) parmi le peroxyde d'hydrogène et/ou un ou plusieurs système(s) générateur(s) de peroxyde d'hydrogène, de préférence le peroxyde d'hydrogène ; et
- une ou plusieurs gommes de scléroglucane, de préférence en une teneur totale en poids supérieure ou égale à 0,5% par rapport au poids total de la composition (B) ;

la composition oxydante (B) étant mélangée avec la composition colorante (A) juste avant l'emploi (ie application sur lesdites fibres) (extemporanément).

L'invention a également pour objet un dispositif (ou " kit ") à plusieurs compartiments permettant la mise en œuvre du procédé de coloration des fibres kératiniques, de préférence comprenant au moins deux compartiments, un premier compartiment contenant la composition colorante (A) telle que définie précédemment, et le second compartiment contenant la composition oxydante (B) telle que définie précédemment les compositions des compartiments étant destinées à être mélangées avant application, pour donner la formulation après mélange ; en particulier le kit peut être un dispositif aérosol.

Au sens de la présente invention, par « composition pour la coloration » ou par « composition colorante », on entend une composition destinée à être appliquée sur les fibres kératiniques, de préférence les fibres kératiniques humaines et en particulier les cheveux, en particulier après mélange avec une composition oxydante (B) telle que définie précédemment comprenant au moins un agent oxydant chimique.

Au sens de la présente invention, par « composition colorante prête à l'emploi » ou « composition prête à l'emploi », on entend une composition résultant du mélange d'une composition colorante et d'une composition oxydante, destinée à être appliquée immédiatement sur les fibres kératiniques. La composition colorante prête à l'emploi est avantageusement préparée juste avant l'application sur lesdites fibres kératiniques.

Au sens de la présente invention, par « extemporané » ou « extemporanément » on entend notamment moins de 30 minutes, de préférence moins de 15 minutes avant l'application sur les fibres kératiniques, de préférence moins de 5 minutes. En particulier, le mélange est appliqué immédiatement après sa préparation. Le procédé selon l'invention permett ainsi de conduire à de très bonnes performances de coloration des fibres kératiniques, notamment en termes

de montée, d'intensité, de chromaticité et/ou de sélectivité. Le procédémet en œuvre des compositions ayant de bonnes propriétés rhéologiques pour ne pas couler, lors de leur application, sur le visage, le cuir chevelu, ou en dehors des zones que l'on se propose de teindre.

Les compositions mises en œuvre dans le procédé selon l'invention sont stables. Par « stable » au sens de la présente invention on entend en particulier que des caractéristiques physiques telles que l'aspect, le pH et/ou la viscosité varient peu, voire ne varient pas, au court du temps, en particulier, que la viscosité de la composition n'évolue pas ou peu pendant le stockage et/ou que la composition ne déphase pas au cours du stockage. En particulier, il est souhaitable que les compositions de coloration soient stables dans les temps, en particulier stables après 1 mois à 45°C, voire après 2 mois à 45°C.

De plus les compositions mises en œuvre dans le procédé selon l'invention présentent l'avantage d'être stables (ne déphasent pas) indépendamment du pH et en particulier vis-à-vis de pH extrêmes (par exemple à des pH alcalins allant de 9 à 12). Enfin, les compositions sont de préférence stables (ne déphasent pas) même en présence d'une teneur importante en certains composés, comme par exemple en colorants d'oxydation et/ou des composés cationiques tels que des polymères cationiques.

Par ailleurs, les compositions mises en œuvre dans le procédé selon l'invention sont de façon avantageuse translucides, ce qui leur confère un aspect visuel esthétique et attrayant pour le consommateur, de même que la composition prête à l'emploi résultant du mélange des compositions colorantes (A) et oxydante (B). Ce produit de coloration translucide offre la possibilité de visualiser l'évolution du résultat couleur pendant le temps de pause du produit laissant le choix de stopper au moment où le résultat lui convient.

De plus, grâce à leurs propriétés rhéologiques très stables et proches les compositions colorantes et oxydantes mises en œuvre se mélangent particulièrement facilement et permettent éventuellement l'utilisation d'un conditionnement en aérosol pour la mise en œuvre du procédé selon l'invention. D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine, notamment dans les expressions « compris entre » et « allant de... à ... » et « variant de ... à ... » .

Les fibres kératiniques sont de préférence des fibres kératiniques humaines,de préférence les cheveux.

L'expression « *au moins un* » est équivalente à l'expression « *un ou plusieurs* ».

Avantageusement, les compositions colorante (A) et oxydante (B) mises en œuvre dans le procédé selon l'invention présentent une texture épaissie, sous forme de crème ou de gel, et de préférence les compositions (A) et (B) sont translucides.

Les compositions mises en œuvre dans le procédé selon l'invention présentent généralement à température ambiante une viscosité supérieure à 50 cps, de préférence comprise entre 200 et 100000 cps, plus préférentiellement entre 400 et 50000 cps, et encore plus préférentiellement entre 500 et10000 cps, mieux entre 600 et8000 cps. Cette viscosité est mesurée à 25°C à une vitesse de rotation de 200 tours/mn à l'aide d'un rhéomètre tel que le rheomat RM 180 équipé d'un mobile n° 3 ou 4, la mesure étant effectuée après 60 secondes de rotation du mobile (temps au bout duquel on observe une stabilisation de la viscosité et de la vitesse de rotation du mobile.

***Composition colorante (A)***

***Colorants d'oxydation***

La composition colorante (A) selon l'invention comprend un ou plusieurs colorants d'oxydation.

Les précurseurs de colorant d'oxydation utilisables dans la présente invention sont en général choisis parmi les bases d'oxydation, éventuellement combinées à un ou plusieurs coupleurs.

Les bases d'oxydation peuvent être de préférence choisies parmi les paraphénylènediamines, les bis-phénylalkylènediamines, les para-aminophénols, les ortho-aminophénols, les bases hétérocycliques et leurs sels d'addition.

Préférentiellement, la ou les bases d'oxydation de l'invention sont choisies parmi les paraphénylènediamines et les bases hétérocycliques. Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino-N,N-diéthyl-3-méthylaniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino-2-chloroaniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2 hydroxyméthyl paraphénylènediamine, la 2-méthoxyméthyl paraphénylènediamine, la N,N-diméthyl-3-méthyl paraphénylènediamine, la N,N-(éthyl,-β-hydroxyéthyl) paraphénylènediamine, la N-(β,γ-dihydroxypropyl) paraphénylènediamine, la N-(4' aminophényl) paraphénylènediamine, la N-phényl paraphénylènediamine, la 2-β- hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, la 2-méthoxyméthyl paraphénylènediamine et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl)-N,N'-bis-(4'-aminophényl)éthylènediamine, la N,N'-bis-(4-aminophényl) tétraméthylènediamine, la N,N'-bis-(β-hydroxyéthyl)-N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3' méthylphényl) éthylènediamine, le 1,8-bis-(2,5-diaminophénoxy)-3,6-dioxaoctane, et leurs sels d'addition.

Parmi les para-aminophénols, on peut citer à titre d'exemple, le para-amino phénol, le 4-amino-3-méthyl phénol, le 4-amino-3-fluoro phénol, le 4-amino-3-chlorophénol, le 4-amino-3-hydroxyméthyl phénol, le 4-amino-2-méthyl phénol, le 4-amino-2-hydroxyméthyl phénol, le 4-amino-2-méthoxyméthyl phénol, le 4-amino-2-aminométhyl phénol, le 4-amino 2-(β-hydroxyéthyl aminométhyl) phénol, le 4-amino-2-fluoro phénol, et leurs sels d'addition avec un acide.

Parmi les ortho-aminophénols, on peut citer à titre d'exemple, le 2-amino phénol, le 2-amino-5-méthyl phénol, le 2-amino-6-méthyl phénol, le 5-acétamido-2- amino phénol, et leurs sels d'addition.

Parmi les bases hétérocycliques, on peut en particulier les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 3,4-diamino pyridine, et leurs sels d'addition.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308.

A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine; l'acide 3-aminopyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-aminopyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-aminopyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-aminopyrazolo[1,5-a]pyridine-7-ol ; le 2-β-hydroxyéthoxy-3-aminopyrazolo[1,5-a]pyridine; le 2-(4-diméthylpypérazinium-1-yl)-3-aminopyrazolo[1,5-a]pyridine, ainsi que leurs sels d'addition.

Plus particulièrement les bases d'oxydation selon l'invention sont choisies parmi les 3-aminopyrazolo-[1,5 a]-pyridines de préférence substituées en position 2 par :

a) un groupe (di)($C_1$-$C_6$)(alkyl)amino les groupes alkyles pouvant être substitués par un ou plusieurs groupes hydroxy, amino, ou imidazolium ;

b) un groupe hétérocycloalkyle comprenant de 5 à 7 chainons, et de 1 à 3 hétéroatomes, cationique ou non, éventuellement substitué par un ou plusieurs groupes ($C_1$-$C_6$)alkyle tel que di($C_1$-$C_4$)alkylpipérazinium ;

c) un groupe ($C_1$-$C_6$)alkoxy éventuellement substitué par un ou plusieurs groupes hydroxy tel que β-hydroxyalkoxy ainsi que leurs sels d'addition.

Parmi les dérivés pyrimidiniques, on peut citer les composés décrits par exemple dans les brevets DE 2359399 ; JP 88 169571 ; JP 05-63124 ; EP 0770375 ou demande de brevet WO 96/15765 comme la 2,4,5,6-tétra-aminopyrimidine, la 4 hydroxy 2,5,6-triaminopyrimidine, la 2 hydroxy 4,5,6-triaminopyrimidine, la 2,4 dihydroxy 5,6-diaminopyrimidine, la 2,5,6 triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5 diamino 1-méthyl pyrazole, le 4,5-diamino 1-(β-hydroxyéthyl) pyrazole, le 3,4-diamino pyrazole, le 4,5-diamino-1- (4'-chlorobenzyl) pyrazole, le 4,5-diamino-1,3-diméthyl pyrazole, le 4,5-diamino-3-méthyl-1-phényl pyrazole, le 4,5-diamino-1-méthyl-3-phényl pyrazole, le 4-amino-1,3-diméthyl-5-hydrazino pyrazole, le 1-benzyl-4,5-diamino-3-méthyl pyrazole, le 4,5-diamino-3-tert-butyl-1-méthyl pyrazole, le 4,5-diamino-1-tert-butyl 3-méthyl pyrazole, le 4,5-diamino-1-(β-hydroxyéthyl)-3-méthyl pyrazole, le 4,5-diamino-1-éthyl-3-méthyl pyrazole, le 4,5-diamino-1-éthyl-3-(4'-méthoxyphényl) pyrazole, le 4,5-diamino-1-éthyl 3-hydroxyméthyl pyrazole, le 4,5-diamino-3-hydroxyméthyl-1-méthyl pyrazole, le 4,5-diamino-3-hydroxyméthyl-1-isopropyl pyrazole, le 4,5-diamino-3-méthyl-1-isopropyl pyrazole, le 4-amino-5-(2'-aminoéthyl)amino-1,3-diméthyl pyrazole, le 3,4,5-triamino pyrazole, le 1-méthyl-3,4,5-triamino pyrazole, le 3,5-diamino-1-méthyl-4-méthylamino pyrazole, le 3,5-diamino-4-(β-hydroxyéthyl)amino-1-méthyl pyrazole, et leurs sels d'addition. De préférence les bases d'oxydation hétérocycliques de l'invention sont choisies parmi les 4,5-diaminopyrazoles telle que le 4,5-diamino-1-(β-hydroxyéthyl) pyrazole. On peut aussi utiliser le 4-5-diamino-1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un de ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La ou les bases d'oxydation utilisées dans le cadre de l'invention sont en général présentes en quantité allant de 0,001 à 10 % en poids environ du poids total de la composition colorante, de préférence allant de 0,005 à 5 %.

Les coupleurs additionnels conventionnellement utilisés pour la teinture de fibres kératiniques sont de préférence choisis parmi les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro-1,3-dihydroxy benzène, le 1-hydroxy-3-aminobenzène, le 2-méthyl-5-aminophénol, le 3-amino-2-chloro-6-méthyl phénol, le 2-méthyl-5-hydroxyéthylaminophénol, le 2,4-diamino-1-(β-hydroxyéthyloxy) benzène, le 2-amino-4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le thymol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H-3-méthyl pyrazole-5-one, la 1-phényl-3-méthyl pyrazole 5-one, le 2,6-diméthyl pyrazolo-[1,5-b]-1,2,4-triazole, le 2,6-diméthyl-[3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Dans le cadre de la présente invention, lorsqu'ils sont présents, le ou les coupleurs sont généralement présents en quantité totale allant de 0,001 à 10 % en poids environ du poids total de la composition colorante, de préférence allant de 0,005 à 5 % en poids par rapport au poids total de la composition colorante (A).

De préférence, la teneur totale en colorants d'oxydation dans la composition selon l'invention est comprise entre 0,001 à 20% en poids ; de préférence entre 0,001% et 10% en poids, de préférence entre 0,01% et 5% en poids, par rapport au poids de la composition colorante (A).

*Gommes de scléroglucane*

Selon l'invention, la composition (A) comprend une ou plusieurs gommes de scléroglucane, en une teneur totale supérieure ou égale à 0,5% en poids par rapport au poids de la composition (A).

Les gommes de scléroglucane sont des polysaccharides d'origine microbienne produits par un champignon de type Sclerotium, en particulier Sclerotium rolfsii. Ce sont des polysaccharides constitués uniquement de motifs glucose.

Les gommes de scléroglucane peuvent être modifiées ou non. De préférence les gommes de scléroglucane

utilisées dans la présente invention sont non modifiées.

Des exemples de gommes de scléroglucane utilisables dans la présente invention sont, de manière non limitative, les produits vendus sous la dénomination ACTIGUM CS, en particulier ACTIGUM CS 11, par la société SANOFI BIO INDUSTRIES et sous la dénomination AMIGUM ou AMIGEL par la société ALBAN MULLER INTERNATIONAL.

D'autres gommes de scléroglucane, telles que celle traitée au glyoxal décrite dans la demande de brevet français n° 2 633 940, peuvent également être utilisées.

La ou les gommes de scléroglucane utilisables selon l'invention représentent de préférence de 0,5 à 10 % en poids, plus préférentiellement de 0,5 à 5 % en poids, et encore plus préférentiellement de 0,5 à 3 % en poids, mieux de 0,5% à 2% en poids et encore plus préférentiellement de 0,7 à 1,5 %en poids, par rapport au poids total de la composition (A).

***Acides carboxyliques***

La composition colorante (A) selon l'invention peut avantageusement comprendre un ou plusieurs acides carboxyliques, et/ou leurs sels d'addition et/ou leurs solvates, ledit ou lesdits acides carboxyliques étant des composés aliphatiques, comprenant de 2 à 10 atomes de carbone et comprenant de préférence au moins deux groupements carboxyliques.

De façon préférés, ils sont choisis parmi les acides dicarboxyliques et/ou tricarboxyliques aliphatiques comprenant de 2 à 10 atomes de carbone, de préférence de 2 à 8 atomes de carbone, mieux de 2 à 6 atomes de carbone.

En particulier, le ou les acides carboxyliques est/sont saturés ou insaturés, substitués ou non substitués.

De façon préférée, le ou les acides carboxyliques sont choisis parmi les composés de formule suivante :

$$XO_2C\text{-}[R]_n\text{-}CO_2X'$$

dans laquelle:

- n vaut 0 ou 1
- R est un radical aliphatique en $C_1$-$C_8$, de préférence en $C_1$-$C_6$, cyclique ou acyclique, de préférence acyclique, saturé ou insaturé, linéaire ou ramifié, comprenant entre 0 et 7 insaturations, (doubles et/ou triples liaisons) et/ou 0 à 2 cycles et optionnellement substitué par un ou plusieurs substituants choisis parmi les groupements hydroxy (-OH), et/ou carboxy (-COOX") ;

    - X et X', X" représentent indépendamment un atome d'hydrogène, un sel d'ammonium, un sel de métal alcalin, tel que Li, Na, K, ou un sel d'alcalino-terreux tel que Be, Mg, Ca ou un sel dérivé d'une amine organique telle qu'une alkylamine.

De façon préférée dans la formule ci-dessus, n vaut 0 ou 1, et R est un radical aliphatique en $C_1$-$C_6$, mieux en $C_1$-$C_4$, mieux encore en $C_1$-$C_3$, acyclique, saturé ou insaturé, linéaire ou ramifié, comprenant entre 0 et 2 insaturations, (doubles et/ou triples liaisons) optionnellement substitué par un ou plusieurs substituants choisis parmi les groupements hydroxy (-OH), et/ou carboxy (-COOX");

- X et X', X" représentent indépendamment un atome d'hydrogène, un sel d'ammonium, un sel de métal alcalin atome, tel que Li, Na, K, ou un sel d'alcalino-terreux tel que Be, Mg, Ca ou un sel dérivé d'une amine organique telle qu'une alkylamine.

Encore plus préférentiellement, dans la formule ci-dessus, n vaut 0 ou 1 et R est un radical aliphatique en $C_1$-$C_4$, acyclique, saturé ou insaturé, linéaire ou ramifié, alkyl ou alcényl comprenant de 0 à 1 insaturation, optionnellement substitué par un ou plusieurs substituants choisis parmi les groupements hydroxy (-OH), et/ou carboxy (-COOX"). De préférence n vaut 1.

Par « radical aliphatique » on entend un radical hydrocarboné non aromatique, saturé ou insaturé pouvant être un hydrocarbures à chaîne ouverte (linéaire ou ramifiée) ou un radical alicycliques (ie comprenant un ou plusieurs cycles non-aromatiques, et éventuellement être substitués par un ou plusieurs groupes carboxy, et/ou hydroxy.

Le ou les acides carboxyliques étant aliphatiques ils ne comprennent donc pas de cycle aromatique.

De préférence, les acides carboxyliques peuvent être choisis parmi l'acide oxalique, l'acide malonique, l'acide malique, l'acide glutarique, l'acide citraconique, l'acide citrique, l'acide maléique, l'acide succinique, l'acide adipique, l'acide tartrique, l'acide fumarique, et leurs mélanges.

De façon préférée, le ou les acides carboxyliques comprennent au moins deux groupes carboxyliques et sont choisis parmi l'acide malonique, l'acide citrique, l'acide maléique, l'acide glutarique, l'acide succinique et leurs mélanges ;

de préférence choisis parmi l'acide malonique, l'acide citrique, l'acide maléique,et leurs mélanges.

De manière plus particulièrement préférée, l'acide carboxylique est l'acide citrique.

La teneur totale en acide(s) carboxylique(s) et/ou leurs sels d'addition et/ou leurs solvates varie de préférence de 0.1% à 20% en poids, par rapport au poids total de la composition (A).

De préférence, la teneur totale en acide(s) carboxylique(s) varie de 0,1 à 20, préférentiellement de 0.5 % à 10% en poids, mieux de 1% à 7% en poids, par rapport au poids total de la composition, mieux encore de 2 à 5 % en poids par rapport au poids total de la composition (A).

## Agents alcalins

La composition (A) mise en œuvre dans le procédé selon l'invention comprend un ou plusieurs agents alcalins. Le ou les agents alcalins (également appelés agents alcalinisants) peuvent être minéraux, organiques et/ou hybrides, en particulier minéraux et/ou organiques.

Selon un premier mode de réalisation avantageux de l'invention, le ou les agent(s) alcalin(s) sont choisis parmi le ou les agents alcalins minéraux, de préférence choisis parmi l'ammoniaque, encore appelé hydroxyde d'ammonium, (ou agents précurseurs d'ammoniac comme les sels d'ammonium par exemple les halogénures d'ammonium et en particulier le chlorure d'ammonium) les silicates, les phosphates, les carbonates ou bicarbonates de métaux alcalins ou alcalino-terreux, tels que métasilicates de métaux alcalins ou alcalino-terreux, les carbonates ou bicarbonates de sodium ou de potassium, les hydroxydes de sodium ou de potassium, ou leurs mélanges.

De façon préférée selon ce mode de réalisation, les agents alcalins sont choisis parmi l'ammoniaque (ou agents précurseurs d'ammoniac comme les sels d'ammonium tels que les halogénures d'ammonium, en particulier le chlorure d'ammonium) et/ou les métasilicates. de métaux alcalins ou alcalino-terreux

Selon un second mode de réalisation avantageux de l'invention, le ou les agent(s) alcalin(s) sont choisis parmi le ou les agents alcalins organiques, de préférence choisis parmi les amines organiques dont le $pK_b$ à 25°C est inférieur à 12, et de préférence inférieur à 10, encore plus avantageusement inférieur à 6. Il est à noter qu'il s'agit du $pK_b$ correspondant à la fonction de basicité la plus élevée. En outre, les amines organiques ne comprennent pas de chaîne grasse, alkyle ou alcényle, comprenant plus de dix atomes de carbone.

Le ou les agents alcalins organiques sont par exemple choisis parmi les alcanolamines, les éthylènediamines oxyéthylénées et/ou oxypropylénées, les acides aminés et les composés de formule (XII) suivante :

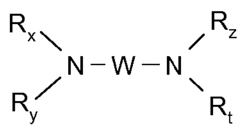

(XII)

dans laquelle, W est un radical divalent alkylène en $C_1$ à $C_6$ éventuellement substitué par un ou plusieurs groupements hydroxyle ou un radical alkyle en $C_1$ à $C_6$, et/ou éventuellement interrompu par un ou plusieurs hétéroatomes tel que O, ou $NR_u$; $R_x$, $R_y$, $R_z$, $R_t$, $R_u$ et identiques ou différents, représentent un atome d'hydrogène, un radical alkyle en $C_1$ à $C_6$ ou hydroxyalkyle en $C_1$ à $C_6$, aminoalkyle en $C_1$ à $C_6$.

On peut citer à titre d'exemple d'amines de formule (XII), le 1,3-diaminopropane, le 1,3-diamino-2-propanol, la spermine, la spermidine.

Selon un mode de réalisation préféré, le ou les agents alcalins organiques sont choisis parmi les alcanolamines et/ou les acides aminés.

Selon un premier mode de réalisation préféré, le ou les agents alcalins sont choisis parmi les alcanolamines.

Par alcanolamine on entend une amine organique comprenant une fonction amine primaire, secondaire ou tertiaire, et un ou plusieurs groupements alkyle, linéaires ou ramifiés, en $C_1$ à $C_8$ porteurs d'un ou plusieurs radicaux hydroxy.

Conviennent en particulier à la réalisation de l'invention les amines organiques choisies parmi les alcanolamines telles que les mono-, di- ou tri-alcanolamines, comprenant un à trois radicaux hydroxyalkyle, identiques ou non, en $C_1$ à $C_4$.

Les composés de ce type, sont de préférence choisis parmi la monoéthanolamine (MEA), la diéthanolamine, la triéthanolamine, la monoisopropanolamine, la diisopropanolamine, la N,N-diméthyléthanolamine, le 2-amino-2-méthyl-1-propanol, la triisopropanol-amine, le 2-amino-2-méthyl-1,3-propanediol, le 3-amino-1,2-propanediol, le 3-diméthylamino-1,2-propanediol, le tris-hydroxyméthylamino-méthane, et leurs mélanges, de préférence la monoéthanolamine (MEA).

Selon un second mode de réalisation préféré, le ou les agents alcalins sont choisis parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides

carboxyliques, sulfoniques, phosphoniques ou phosphoriques. Les acides aminés peuvent se trouver sous forme neutre ou ionique.

A titre d'acides aminés utilisables dans la composition selon la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont choisis parmi les acides aminés basiques, notamment comprenant une fonction amine supplémentaire éventuellement incluse dans un cycle ou dans une fonction uréido.

De tels acides aminés basiques sont choisis de préférence parmi ceux répondant à la formule (XIII) suivante, ainsi que leurs sels

$$R\text{-}CH_2\text{-}CH(NH_2)\text{-}C(O)\text{-}OH \qquad (XIII)$$

dans laquelle, R représente un groupe choisi parmi imidazolyle, de préférence imidazolyl-4-yl ; aminopropyle ; aminoéthyle ;

$$-(CH_2)_2N(H)\text{-}C(O)\text{-}NH_2 \text{ ; et } -(CH_2)_2\text{-}N(H)\text{-}C(NH)\text{-}NH_2.$$

Les composés correspondants à la formule (XIII) sont l'histidine, la lysine, l'arginine, l'ornithine, la citrulline.

L'amine organique peut être aussi choisie parmi les amines organiques de type hétérocycliques. On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la balenine

L'amine organique peut être aussi choisie parmi les composés comportant une fonction guanidine. A titre d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidino-propionique, l'acide 4-guanidinobutyrique et l'acide 2-([amino(imino)méthyl]amino)-éthane-1-sulfonique.

A titre de composés hybrides on peut mentionner les sels des amines citées précédemment avec des acides comme l'acide carbonique, l'acide chlorhydrique.

De préférence, le ou les agents alcalins, présents dans la composition (A) mise en œuvre dans le procédé selon l'invention, sont choisis parmi l'ammoniaque, les alcanolamines, et/ou les acides aminés sous forme neutre ou ionique, en particulier les acides aminés basiques, et de préférence correspondants à ceux de formule (XIII) tels que l'arginine, les métasilicates de métaux alcalins ou alcalinoterreux.

De préférence, la composition selon l'invention comprend un ou plusieurs agents alcalins.

Selon un mode de réalisation particulièrement préféré de l'invention, la composition (A) mise en œuvre dans le procédé selon l'invention comprend :

- un ou plusieurs agent(s) alcalin(s) minéraux, de préférence choisis parmi l'ammoniaque et/ou les métasilicates de métaux alcalins ou alcalinoterreux de préférence l'ammoniaque; et
- un ou plusieurs agents alcalins organiques, de préférence choisis parmi les alcanolamines et/ou les acides aminés, de préférence parmi les alcanolamines, de préférence la monoéthanolamine.

La composition (A) selon l'invention comprend de préférence un ou plusieurs agent(s) alcalin(s), de préférence elle comprend un ou plusieurs agent(s) alcalin(s) minéral(aux) et un ou plusieurs agent(s) alcalin(s) choisi(s) parmi les alcanolamine(s).

Lorsque la composition (A) comprend de l'ammoniaque (hydroxyde d'ammonium), sa teneur va de préférence de 0,1 à 10% en poids, plus préférentiellement de 0,5 à 8% en poids, mieux de 1 à 6% en poids par rapport au poids total de la composition (A).

Lorsque la composition (A) comprend une ou plusieurs alcanolamines, leur teneur totale va de préférence de 0,5 à 10% en poids, plus préférentiellement de 1 à 9% en poids, mieux de 2 à 8% en poids par rapport au poids total de la composition(A).

De façon préférée la composition colorante (A) mise en œuvre dans le procédé selon l'invention comprend une teneur totale en agents alcalins allant de 1 à 20% en poids, plus préférentiellement de 3 à 18% en poids, mieux de 5 à 16 % en poids par rapport au poids total de la composition (A).

**Polymères Associatifs**

La composition colorante (A) mise en œuvre dans le procédé selon l'invention peut également comprendre un ou plusieurs polymères associatifs. Les polymères associatifs selon l'invention sont des polymères comprenant au moins une chaîne grasse en $C_8$ - $C_{30}$ et dont les molécules sont capables, dans le milieu de formulation, de s'associer entre elles ou avec des molécules d'autres composés.

De préférence, la chaîne grasse comporte de 10 à 30 atomes de carbone.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

Les polymères associatifs pouvant être utilisés dans la composition selon l'invention peuvent être choisis parmi les polymères associatifs non ioniques, anioniques, cationiques et amphotères, et leurs mélanges.

De façon particulière, le ou les polymères associatifs sont non ioniques, et de préférence choisis parmi les celluloses modifiées par des groupements comportant au moins une chaîne grasse.
De préférence, le ou les polymères associatifs non ioniques sont choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et les hydroxyéthylcelluloses modifiées par des groupes polyalkylène glycol éther d'alkyl phénol, et leurs mélanges, de préférence la cétylhydroxyéthylcellulose.

Selon un troisième mode de réalisation le ou les polymère(s) associatifs(s) est/sont choisi(s) parmi les polymères associatifs cationiques. Les polymères associatifs de type cationique sont choisis de préférence parmi les dérivés de cellulose quaternisée, les polyacrylates à groupements latéraux aminés non cycliques, les polyuréthanes cationiques, les polyvinyllactames cationiques et le terpolymère acrylique dont la constitution est donnée ci-après.

***Polymères cationiques***

Selon un mode de réalisation avantageux de l'invention, la composition (A) comprend un ou plusieurs polymères cationiques

Comme polymères cationiques utilisables dans les compositions selon l'invention, on peut citer en particulier :

(1) les cyclopolymères d'alkyl diallyl amine ou de dialkyl diallyl ammonium tels que les homopolymères ou copolymères comportant comme constituant principal de la chaîne des motifs répondant aux formules (I) ou (II) :

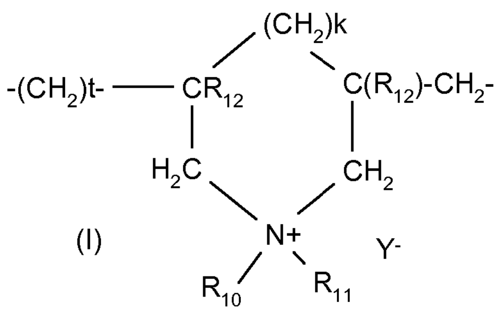
(I)

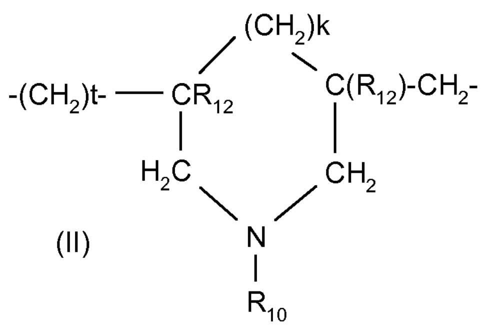
(II)

dans lesquelles

- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle en C1-C6, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11, indépendamment l'un de l'autre, désignent de préférence un groupement alkyle en C1-C4;
- $Y^-$ est un anion tel que bromure, chlorure, acétate, borate, citrate, tartrate, bisulfate, bisulfite, sulfate, phosphate.

On peut citer plus particulièrement l'homopolymère de sels (par exemple chlorure) de diméthyldiallylammonium par exemple vendu sous la dénomination "MERQUAT 100" par la société NALCO, De préférence, les polymères de la

famille (1) sont choisis parmi les homopolymères de dialkyl diallyl ammonium.

(2) les polymères de diammonium quaternaire comprenant des motifs récurrents de formule :

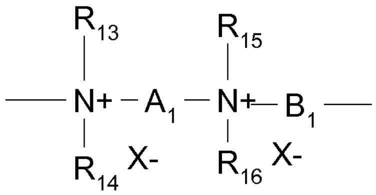
(III)

dans laquelle :

- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- $X^-$ désigne un anion dérivé d'un acide minéral ou organique;
  étant entendu que A1, R13 et R15 peuvent former avec les deux atomes d'azote auxquels ils sont rattachés un cycle pipérazinique ;
  en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désignant :

  a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré de polymérisation défini et unique ou un nombre quelconque de 1 à 4 représentant un degré de polymérisation moyen ;
  b) un reste de diamine bis-secondaire tel qu'un dérivé de pipérazine;
  c) un reste de diamine bis-primaire de formule -NH-Y-NH- où Y désigne un radical hydrocarboné linéaire ou ramifié, ou bien le radical divalent -CH2-CH2-S-S-CH2-CH2- ;
  d) un groupement uréylène de formule -NH-CO-NH- .

De préférence, $X^-$ est un anion tel que le chlorure ou le bromure. Ces polymères ont une masse molaire moyenne en nombre (Mn) généralement comprise entre 1000 et 100000.

On peut citer plus particulièrement les polymères cationiques qui sont constitués de motifs récurrents répondant à la formule :

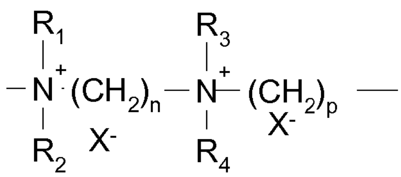
(IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique.

Un composé de formule (IV) particulièrement préféré est celui pour lequel R1, R2, R3 et R4 représentent un radical méthyle, n=3, p=6 et X = Cl, dénommé Hexadimethrine chloride selon la nomenclature INCI (CTFA).

De préférence, le ou les polymère(s) cationique(s) est/sont choisi(s) parmi les homopolymères de dialkyl diallyl ammonium, en particulier les homopolymères de sels de diméthyldiallylammonium, les polymères constitués de motifs récurrents répondant à la formule (IV) ci-dessus, en particulier le poly(dimethyliminio)-1,3-propanediyl(dimethyliminio)-1,6-hexanediyldichloride, dont le nom INCI est hexadimethrine chloride ; et leurs mélanges.
Lorsqu'ils sont présents, la teneur totale en polymères cationiques (différents des polymères associatifs et des polymères fixants) dans la composition (A) peut varier de 0,01 à 10% en poids par rapport au poids de la composition, de préférence de 0,1 à 7 %, par rapport au poids de la composition, encore plus avantageusement de 0,5à 5 % en poids, mieux de 0,5 à 3 % en poids par rapport au poids de la composition (A).

***Tensioactifs***

De préférence, la composition (A) comprend un ou plusieurs agents tensioactifs, ceux-ci peuvent être choisis parmi les agents tensioactifs anioniques, les agents tensioactifs amphotères ou zwittérioniques, les agents tensioactifs non-ioniques et les agents tensioactifs cationiques, et leurs mélanges, de préférence parmi les agents tensioactifs non ioniques, les agents tensioactifs cationiques et leurs mélanges.
On entend par « agent tensioactif anionique », un tensioactif ne comportant à titre de groupements ioniques ou ionisables que des groupements anioniques. Ces groupements anioniques sont choisis de préférence parmi les groupements $CO_2H$, $CO_2^-$, $SO_3H$, $SO_3^-$, $OSO_3H$, $OSO_3^-$, $H_2PO_3$, $HPO_3^-$, $PO_3^{2-}$, $H_2PO_2$, $HPO_2^-$, $PO_2^{2-}$, POH et $PO^-$.
A titre de tensio-actifs, on préfère les tensio-cayifs non ioniques. On peut encore citer les tensioactifs non ioniques de type alkyl(poly)glycoside, notamment représentés par la formule générale suivante :

$$R_1O-(R_2O)_t-(G)_v$$

dans laquelle:

- $R_1$ représente un radical alkyle ou alcényle linéaire ou ramifié comportant 6 à 24 atomes de carbone, notamment 8 à 18 atomes de carbone, ou un radical alkylphényle dont le radical alkyle linéaire ou ramifié comporte 6 à 24 atomes de carbone, notamment 8 à 18 atomes de carbone;
- $R_2$ représente un radical alkylène comportant 2 à 4 atomes de carbone,
- G représente un motif sucre comportant 5 à 6 atomes de carbone,
- t désigne une valeur allant de 0 à 10, de préférence de 0 à 4,
- v désigne une valeur allant de 1 à 15, de préférence de 1 à 4.

De préférence, les tensioactifs alkyl(poly)glycoside sont des composés de formule décrite ci-dessus dans laquelle :

- $R_1$ désigne un radical alkyle saturé ou insaturé, linéaire ou ramifié comportant de 8 à 18 atomes de carbone,
- $R_2$ représente un radical alkylène comportant 2 à 4 atomes de carbone,
- t désigne une valeur allant de 0 à 3, de préférence égale à 0,
- G désigne le glucose, le fructose ou le galactose, de préférence le glucose;
- le degré de polymérisation, c'est-à-dire la valeur de v, pouvant aller de 1 à 15, de préférence de 1 à 4; le degré moyen de polymérisation étant plus particulièrement compris entre 1 et 2.

Les liaisons glucosidiques entre les motifs sucre sont généralement de type 1-6 ou 1-4, de préférence de type 1-4. De préférence, le tensioactif alkyl(poly)glycoside est un tensioactif alkyl(poly)glucoside. On préfère tout particulièrement les alkyl $C_8/C_{16}$-(poly)glucosides 1,4, et notamment les décylglucosides et les caprylyl/capryl glucosides de préférence les caprylyl/capryl glucosides.
Parmi les produits commerciaux, on peut citer les produits vendus par la société COGNIS sous les dénominations PLANTAREN® (600 CS/U, 1200 et 2000) ou PLANTACARE® (818, 1200 et 2000); les produits vendus par la société SEPPIC sous les dénominations ORAMIX CG 110 et ORAMIX® NS 10; les produits vendus par la société BASF sous la dénomination LUTENSOL GD 70 ou encore les produits vendus par la société CHEM Y sous la dénomination AG10 LK.
De préférence, on utilise les alkyl $C_8/C_{16}$-(poly)glycoside 1,4, notamment en solution aqueuse à 53%, tels que ceux commercialisés par COGNIS sous la référence PLANTACARE® 818 UP.
Préférentiellement, les tensioactifs non ioniques sont choisis parmi :

- les alcools gras oxyéthylénés, comportant au moins une chaîne alkyle en $C_8$ à $C_{40}$, notamment en $C_8$-$C_{20}$, encore mieux en $C_{10}$-$C_{18}$ , saturée ou non, linéaire ou ramifiée, et comprenant de 1 à 100 moles d'oxyde d'éthylène, de

préférence de 2 à 50, plus particulièrement de 2 à 40 moles, voire de 3 à 20 moles d'oxyde d'éthylène;, et

- les (alkyl $C_6$-$C_{24}$)(poly)glycosides, et plus particulièrement les (alkyl $C_8$-$C_{18}$)(poly)glycosides ;
- et leurs mélanges ;

et plus préférentiellement encore parmi les (alkyl $C_6$-$C_{24}$)(poly)glycosides, préférentiellement les (alkyl $C_8$-$C_{18}$)(poly)glycosides.

Selon un mode de réalisation préféré de l'invention, la composition (A) comprend un ou plusieurs tensioactifs non ioniques, de préférence choisis parmi les alkyl(poly)glycosides. De préférence la composition selon l'invention comprend un ou plusieurs tensioactifs choisi parmi les (alkyl $C_6$-$C_{24}$)(poly)glycosides, plus préférentiellement parmi les (alkyl $C_8$-$C_{18}$)(poly)glycosides, de préférence parmi les alkyl $C_8$/$C_{16}$-(poly)glucosides, de préférence de type 1,4, et de préférence choisi parmi les décylglucosides et/ou les caprylyl/capryl glucosides et/ou les cocoglucosides.

Selon un premier mode de réalisation, le ou les agents tensioactifs sont non ioniques, de préférence choisis parmi les (alkyl en $C_6$-$C_{24}$)polyglycosides.

Selon un mode de réalisation préféré, la composition (A) comprend au moins un ou plusieurs agents tensioactifs cationiques. De préférence, le ou les tensioactifs cationiques sont choisis parmi les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :

- ceux répondant à la formule générale (X) suivante :

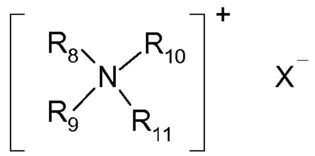

(X)

dans laquelle les groupes $R_8$ à $R_{11}$, qui peuvent être identiques ou différents, représentent un groupe aliphatique, linéaire ou ramifié, comportant de 1 à 30 atomes de carbone, ou un groupe aromatique tel que aryle ou alkylaryle, au moins un des groupes $R_8$ à $R_{11}$ comportant de 8 à 30 atomes de carbone, de préférence de 12 à 24 atomes de carbone. Les groupes aliphatiques peuvent comporter des hétéroatomes tels que notamment l'oxygène, l'azote, le soufre et les halogènes.

Les groupes aliphatiques sont par exemple choisis parmi les groupes alkyle en $C_1$-$C_{30}$, alcoxy en $C_1$-$C_{30}$, polyoxyalkylène ($C_2$-$C_6$), alkylamide en $C_1$-$C_{30}$, alkyl($C_{12}$-$C_{22}$)amidoalkyle($C_2$-$C_6$), alkyl($C_{12}$-$C_{22}$)acétate, et hydroxyalkyle en $C_1$-$C_{30}$, $X^-$ est un anion choisi dans le groupe des halogénures, phosphates, acétates, lactates, alkyl($C_1$-$C_4$)sulfates, alkyl($C_1$-$C_4$)- ou alkyl($C_1$-$C_4$)aryl-sulfonates.

Parmi les sels d'ammonium quaternaire de formule (X), on préfère d'une part, les chlorures de tétraalkylammonium comme, par exemple, les chlorures de dialkyldiméthylammonium ou d'alkyltriméthylammonium dans lesquels le groupe alkyle comporte environ de 12 à 22 atomes de carbone, en particulier les chlorures de béhényltriméthylammonium, de distéaryldiméthylammonium, de *cétyltriméthylammonium,* de benzyldiméthylstéarylammonium ou encore, d'autre part, le méthosulfate de distéaroyléthylhydroxyéthylméthylammonium, le méthosulfate de dipalmitoyléthylhydroxyéthylammonium ou le méthosulfate de distéaroyléthylhydroxyéthylammonium, ou encore, enfin, le chlorure de palmitylamidopropyltriméthylammonium ou le chlorure de stéaramidopropyldiméthyl-(myristylacétate)-ammonium commercialisé sous la dénomination CERAPHYL® 70 par la société VAN DYK.

- les sels d'ammonium quaternaire de l'imidazoline, comme par exemple ceux de formule (XI) suivante :

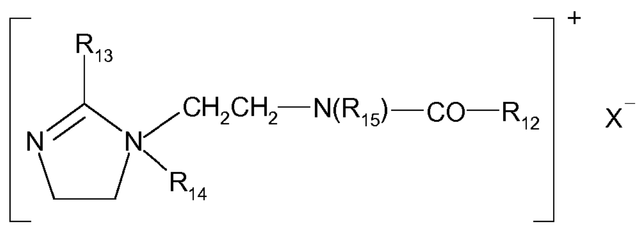

(XI)

dans laquelle

$R_{12}$ représente un groupe alcényle ou alkyle comportant de 8 à 30 atomes de carbone, par exemple dérivés des acides gras du suif,
$R_{13}$ représente un atome d'hydrogène, un groupe alkyle en $C_1$-$C_4$ ou un groupe alcényle ou alkyle comportant de 8 à 30 atomes de carbone,
$R_{14}$ représente un groupe alkyle en $C_1$-$C_4$,
$R_{15}$ représente un atome d'hydrogène, un groupe alkyle en $C_1$-$C_4$, $X^-$ est un anion choisi dans le groupe des halogénures, phosphates, acétates, lactates, alkyl($C_1$-$C_4$)sulfates, alkyl($C_1$-$C_4$)- ou alkyl($C_1$-$C_4$)aryl-sulfonates.

De préférence, $R_{12}$ et $R_{13}$ désignent un mélange de groupes alcényle ou alkyle comportant de 12 à 21 atomes de carbone, par exemple dérivés des acides gras du suif, $R_{14}$ désigne un groupe méthyle, $R_{15}$ désigne un atome d'hydrogène. Un tel produit est par exemple commercialisé sous la dénomination REWOQUAT® W 75 par la société REWO.

- les sels de di- ou de triammonium quaternaire en particulier de formule (XII) suivante :

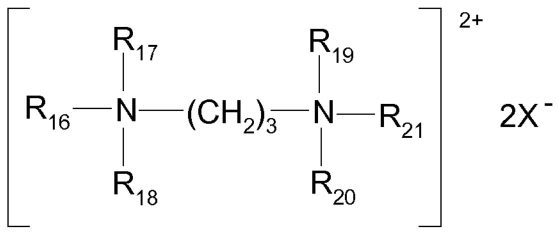

(XII)

dans laquelle $R_{16}$ désigne un groupe alkyle comportant environ de 16 à 30 atomes de carbone éventuellement hydroxylé et/ou interrompu par un ou plusieurs atomes d'oxygène,

$R_{17}$ est choisi parmi l'hydrogène ou un groupe alkyle comportant de 1 à 4 atomes de carbone ou un groupe $-(CH_2)_3-N^+(R_{16a})(R_{17a})(R_{18a})$,
$R_{16a}$, $R_{17}$a, $R_{18}$a, $R_{18}$, $R_{19}$, $R_{20}$ et $R_{21}$, identiques ou différents, sont choisis parmi l'hydrogène ou un groupe alkyle comportant de 1 à 4 atomes de carbone, et
$X^-$ est un anion choisi dans le groupe des halogénures, acétates, phosphates, nitrates, alkyl($C_1$-$C_4$)sulfates, alkyl($C_1$-$C_4$)- ou alkyl($C_1$-$C_4$)aryl-sulfonates, en particulier méthylsulfate et éthylsulfate.

De tels composés sont par exemple le Finquat CT-P proposé par la société FINETEX (Quaternium 89), le Finquat CT proposé par la société FINETEX (Quaternium 75).

- les sels d'ammonium quaternaire contenant une ou plusieurs fonctions esters, tels que, par exemple, ceux de formule (XIII) suivante

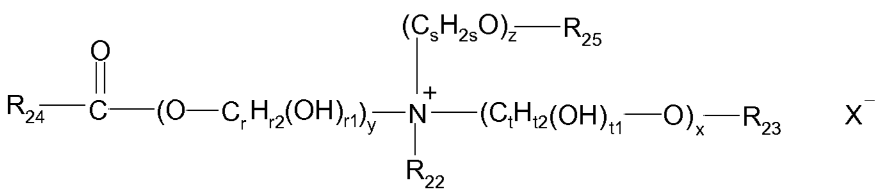

(XIII)

dans laquelle :

$R_{22}$ est choisi parmi les groupes alkyles en $C_1$-$C_6$ et les groupes hydroxyalkyle ou dihydroxyalkyle en $C_1$-$C_6$,
$R_{23}$ est choisi parmi :

- le groupe

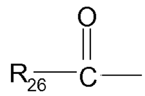,

- les groupes $R_{27}$ hydrocarbonés en $C_1$-$C_{22}$, linéaires ou ramifiés, saturés ou insaturés,
- l'atome d'hydrogène,

$R_{25}$ est choisi parmi :

- le groupe

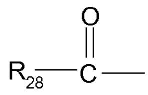,

- les groupes $R_{29}$ hydrocarbonés en $C_1$-$C_6$, linéaires ou ramifiés, saturés ou insaturés,
- l'atome d'hydrogène,

$R_{24}$, $R_{26}$ et $R_{28}$, identiques ou différents, sont choisis parmi les groupes hydrocarbonés en $C_7$-$C_{21}$, linéaires ou ramifiés, saturés ou insaturés,
r, s et t, identiques ou différents, sont des entiers valant de 2 à 6,
r1 et tl, identiques ou différents, valent 0 ou 1,
r2 + r1 = 2 r et t1 + t2 = 2 t,
y est un entier valent de 1 à 10,
x et z, identiques ou différents, sont des entiers valant de 0 à 10,
$X^-$ est un anion simple ou complexe, organique ou inorganique,
sous réserve que la somme x + y + z vaut de 1 à 15, que lorsque x vaut 0 alors $R_{23}$ désigne $R_{27}$ et que lorsque z vaut 0 alors $R_{25}$ désigne $R_{29}$.

Les groupes alkyles $R_{22}$ peuvent être linéaires ou ramifiés et plus particulièrement linéaires.

De préférence, $R_{22}$ désigne un groupe méthyle, éthyle, hydroxyéthyle ou dihydroxypropyle, et plus particulièrement un groupe méthyle ou éthyle.

Avantageusement, la somme x + y + z vaut de 1 à 10.

Lorsque $R_{23}$ est un groupe $R_{27}$ hydrocarboné, il peut être long et avoir de 12 à 22 atomes de carbone, ou court et avoir de 1 à 3 atomes de carbone.

Lorsque $R_{25}$ est un groupe $R_{29}$ hydrocarboné, il a de préférence 1 à 3 atomes de carbone.

Avantageusement, $R_{24}$, $R_{26}$ et $R_{28}$, identiques ou différents, sont choisis parmi les groupes hydrocarbonés en $C_{11}$-$C_{21}$, linéaires ou ramifiés, saturés ou insaturés, et plus particulièrement parmi les groupes alkyle et alcényle en $C_{11}$-$C_{21}$, linéaires ou ramifiés, saturés ou insaturés.

De préférence, x et z, identiques ou différents, valent 0 ou 1. Avantageusement, y est égal à 1.

De préférence, r, s et t, identiques ou différents, valent 2 ou 3, et encore plus particulièrement sont égaux à 2.

L'anion $X^-$ est de préférence un halogénure, de préférence chlorure, bromure ou iodure, un alkyl($C_1$-$C_4$)sulfate, alkyl($C_1$-$C_4$)- ou alkyl($C_1$-$C_4$)aryl-sulfonate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion $X^-$ est encore plus particulièrement le chlorure, le méthylsulfate ou l'éthylsulfate.

On utilise plus particulièrement dans la composition selon l'invention, les sels d'ammonium de formule (XIII) dans laquelle :

- $R_{22}$ désigne un groupe méthyle ou éthyle,
- x et y sont égaux à 1,
- z est égal à 0 ou 1,
- r, s et t sont égaux à 2,
- $R_{23}$ est choisi parmi :
- le groupe

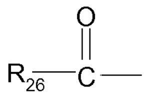,

- les groupes méthyle, éthyle ou hydrocarbonés en $C_{14}$-$C_{22}$,
- l'atome d'hydrogène,
- $R_{25}$ est choisi parmi :
- le groupe

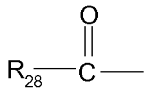,

- l'atome d'hydrogène,
- $R_{24}$, $R_{26}$ et $R_{28}$, identiques ou différents, sont choisis parmi les groupes hydrocarbonés en $C_{13}$-$C_{17}$, linéaires ou ramifiés, saturés ou insaturés, et de préférence parmi les groupes alkyle et alcényle en $C_{13}$-$C_{17}$, linéaires ou ramifiés, saturés ou insaturés.

Avantageusement, les groupes hydrocarbonés sont linéaires.

On peut citer par exemple parmi les composés de formule (XIII) les sels, notamment le chlorure ou le méthylsulfate de diacyloxyéthyldiméthylammonium, de diacyloxyéthylhydroxyéthyl méthylammonium, de monoacyloxyéthyldihydroxyéthylméthyl ammonium, de triacyloxyéthylméthylammonium, de mono acyloxyéthylhydroxyéthyldiméthylammonium, et leurs mélanges. Les groupes acyles ont de préférence 14 à 18 atomes de carbone et proviennent plus particulièrement d'une huile végétale comme l'huile de palme ou de tournesol. Lorsque le composé contient plusieurs groupes acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation, tel qu'un halogénure d'alkyle, de préférence de méthyle ou d'éthyle, un sulfate de dialkyle, de préférence de méthyle ou d'éthyle, le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention peut contenir par exemple un mélange de sels de mono-, di- et triester d'ammonium quaternaire avec une majorité en poids de sels de diester.

On peut aussi utiliser les sels d'ammonium contenant au moins une fonction ester décrits dans les brevets US-A-4874554 et US-A-4137180.

On peut également utiliser le chlorure de béhénoylhydroxypropyltriméthylammonium, par exemple, proposé par la société KAO sous la dénomination Quartamin BTC 131.

De préférence, les sels d'ammonium contenant au moins une fonction ester contiennent deux fonctions esters.

De préférence, le ou les agents tensioactifs cationiques, sont choisis parmi les sels de cétyltriméthylammonium, de béhényltriméthylammonium, de dipalmitoyléthylhydroxy éthylméthylammonium, et leurs mélanges, et plus particulièrement le chlorure de béhényltriméthylammonium, le chlorure de cétyltriméthylammonium, le méthosulfate de dipalmitoyléthylhydroxy éthylammonium, et leurs mélanges.

La composition (A) comprend, de préférence un ou plusieurs agents tensioactifs en une teneur totale allant de 0,01 à 20 % en poids, plus préférentiellement de 0,05 à 10 % en poids, mieux de 0,1 à 5 % en poids, par rapport au poids total de la composition (A).

La composition (A) comprend, de préférence un ou plusieurs agents tensioactifs non ioniques en une teneur totale allant de 0,01 à 10 % en poids, plus préférentiellement de 0,05 à 5 % en poids, mieux de 0,1 à 3 % en poids, par rapport au poids total de la composition (A).

La composition (A) comprend de préférence un ou plusieurs agents tensioactifs cationiques en une teneur totale allant de 0,01 à 10 % en poids, plus préférentiellement de 0,05 à 5 % en poids, mieux de 0,1 à3 % en poids, par rapport au poids total de la composition (A).

De manière préférée, le ou les agents tensioactifs sont choisis parmi les tensioactifs cationiques ou non ioniques, et leurs mélanges, préférentiellement cationiques. De préférence la composition (A) comprend au moins un ou plusieurs

tensioactifs cationiques et un ou plusieurs tensioactifs non ioniques.

## Composition Oxydante (B)

**Agent oxydant** :

La composition oxydante (B) mise en œuvre dans le procédé selon l'invention contient un ou plusieurs agent(s) oxydant(s) chimique(s), de préférence choisi(s) parmi le peroxyde d'hydrogène et/ou un ou plusieurs système(s) générateur(s) de peroxyde d'hydrogène.

Par « agent oxydant chimique » on entend un agent oxydant différent de l'oxygène de l'air.

De préférence, le ou les agents oxydants chimiques sont choisis parmi le peroxyde d'hydrogène, les sels peroxygénés comme par exemple les persulfates, les perborates, les peracides et leurs précurseurs, les percarbonates de métaux alcalins ou alcalino-terreux tel que le peroxyde de carbonate de sodium ou appelé percarbonate de sodium et les peracides et leurs précurseurs ; les bromates ou ferricyanures de métaux alcalins, les agents oxydants chimiques solides générateurs de peroxyde d'hydrogène tels que le peroxyde d'urée et les complexes polymériques pouvant libérer du peroxyde d'hydrogène notamment ceux comprenant un monomère hétérocyclique vinylique tels que les complexes polyvinylpyrrolidone/$H_2O_2$ en particulier se présentant sous forme de poudres ; les oxydases produisant du peroxyde d'hydrogène en présence d'un substrat adéquat (par exemple le glucose dans le cas de glucose oxydase ou l'acide urique avec l'uricase).

De manière préférée, le ou les agents oxydants chimiques sont choisis parmi le peroxyde d'hydrogène, le peroxyde d'urée, les bromates ou ferricyanures de métaux alcalins, les sels peroxygénés, et les mélanges de ces composés,

De manière particulièrement préférée, l'agent oxydant chimique est du peroxyde d'hydrogène.

De manière préférée, le ou les agents oxydants chimiques représentent de 0,05 à 40 % en poids, de préférence de 0,5 à 30 % en poids, plus préférentiellement de 1 à 20 % en poids, et mieux de 1,5 à 15 % en poids par rapport au poids total de la composition oxydante (B).

De préférence, la composition oxydante (B)selon l'invention ne contient pas de sels peroxygénés.

### Gomme de scléroglucane

Comme indiqué précédemment, la composition oxydante (B) comprend une ou plusieurs gommes de scléroglucane, de préférence en une teneur totale supérieure ou égale à 0,5% en poids par rapport au poids de la composition.

De préférence, la ou les gommes de scléroglucane utilisables selon l'invention représentent de préférence de 0,5 à 10 % en poids, plus préférentiellement de 0,5 à 5 % en poids, et encore plus préférentiellement de 0,5 à 3 % en poids, voire de 0,7 à 2% en poids, par rapport au poids total de la composition oxydante (B).

### Agents séquestrants phosphorés

De préférence selon l'invention, la composition (B) comprend un ou plusieurs agents séquestrants phosphorés.

La définition d'un « agent séquestrant » (ou « agent chélatant »), est bien connu de l'homme du métier et fait référence à un composé ou un mélange de composés capable(s) de former un chélate avec un ion métallique. Un chélate est un complexe inorganique dans lequel un composé (l'agent séquestrant ou chélatant) est coordiné à un ion métallique, c'est-à-dire qu'il forme une ou plusieurs liaisons avec l'ion métallique (formation d'un cycle incluant l'ion métallique).

Un agent séquestrant (ou chélatant) comprend généralement au moins deux atomes donneurs d'électrons qui permettent la formation de liaisons avec l'ion métallique.

Dans le cadre de la présente invention, le ou les agents séquestrants sont des agents séquestrants phosphorés, c'est-à-dire des agents séquestrants qui comprennent un ou plusieurs atomes de phosphore, de préférence au moins deux atomes de phosphore.

Le ou les agents séquestrants phosphorés utilisés dans la composition selon l'invention sont de préférence choisis parmi :

- les dérivés phosphorés inorganiques de préférence choisis parmi les phosphates et les pyrophosphates de métaux alcalins ou alcalino-terreux, de préférence de métaux alcalins tels que le pyrophosphate de sodium, le pyrophosphate de potassium, le pyrophosphate de sodium décahydrate ; et les polyphosphates de métaux alcalin ou alcalino-terreux, de préférence de métaux alcalins, tels que le sodium hexamétaphosphate, le sodium polyphosphate, le sodium tripolyphosphate, le sodium trimétaphosphate ; éventuellement hydratés, et leurs mélanges ;
- les dérivés phosphorés organiques, tels que les (poly)phosphates et (poly)phosphonates organiques, comme l'acide

étidronique et/ou ses sels de métaux alcalins ou alcalino-terreux comme le tétrasodium étidronate, et leurs mélanges.

De préférence, le ou les agent(s) séquestrant(s) phosphoré(s) est(sont) choisi(s) parmi les composés linéaires ou cycliques comprenant au moins deux atomes de phosphore liés entre eux de façon covalente par au moins un linker L comprenant au moins un atome d'oxygène et/ou au moins un atome de carbone.

Dans un mode de réalisation, le ou les agent(s) séquestrant(s) phosphoré(s) est(sont) choisi(s) parmi les dérivés phosphorés inorganiques, comprenant de préférence au moins 2 atomes de phosphore. Plus préférentiellement, le ou les séquestrant(s) phosphoré(s) est(sont) choisi(s) parmi les pyrophosphates de métaux alcalins ou alcalino-terreux, mieux parmi les pyrophosphates de métaux alcalins, en particulier le pyrophosphate de sodium (encore appelé tétrasodium pyrophosphate).

Dans un autre mode de réalisation, le ou les agent(s) séquestrant(s) phosphoré(s) est(sont) choisi(s) parmi les dérivés phosphorés organiques, comprenant de préférence au moins 2 atomes de phosphore. Plus préférentiellement, le ou les séquestrant(s) phosphoré(s) est(sont) choisi(s) parmi l'acide étidronique (encore appelé acide 1-hydroxyéthane 1,1-diphosphonique) et/ou ses sels de métaux alcalins ou alcalino-terreux, de préférence de métaux alcalins comme le tétrasodium étidronate.

Ainsi, de manière préférée, le ou les agents séquestrants phosphorés sont choisis parmi les pyrophosphates de métaux alcalins, l'acide étidronique et/ou ses sels de métaux alcalins, et un mélange de ces composés.

De manière particulièrement préférée, le ou les agents séquestrants phosphorés sont choisis parmi le tétrasodium étidronate, l'acide étidronique, le tétrasodium pyrophosphate et un mélange de ces composés.

Le ou les agents séquestrants phosphorés utilisables selon l'invention représentent généralement au moins 0,001 % en poids, de préférence de 0,001 à 5 % en poids, plus préférentiellement de 0,01 à 1 % en poids, et encore plus préférentiellement de 0,01 à 0,5 % en poids, par rapport au poids total de la composition (B).

La composition oxydante (B) peut également renfermer divers composés additionnels ou divers adjuvants utilisés classiquement dans les compositions pour la coloration des cheveux notamment tels que définis précédemment, en particulier tels que un ou plusieurs agents tensioactifs tels que décrits précédemment.

Cette composition oxydante (B) peut également comprendre un ou plusieurs solvants organiques hydrosolubles tels que décrits ci-après.

Enfin, la composition oxydante (B) se présente sous diverses formes, comme par exemple une solution, une émulsion ou un gel.

***Milieu***

Les compositions mises en œuvre selon l'invention sont cosmétiquement acceptables et comprennent en conséquence un milieu cosmétiquement acceptable.

Par « milieu cosmétiquement acceptable », on entend un milieu compatible avec les fibres kératiniques.

Le milieu cosmétiquement acceptable approprié pour la coloration des fibres kératiniques, appelé aussi « support » de coloration, comprend généralement de l'eau ou un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant de 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; le glycérol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants usuels décrits ci-dessus, s'ils sont présents, représentent habituellement de 1 à 40% en poids, plus préférentiellement de 5 à 30% en poids, par rapport au poids total de la composition.

Les compositions mises en œuvre selon l'invention comprennent généralement de l'eau ou un mélange d'eau et d'un ou plusieurs solvants organiques ou un mélange de solvants organiques.

La composition colorante (A) et la composition oxydante (B) selon l'invention comprennent de préférence de l'eau.

De préférence la teneur en eau varie de 5 à 95% en poids, plus préférentiellement de 10 à 90 % en poids, mieux de 20 à 80 % en poids, par rapport au poids total de la composition (A).

La composition oxydante (B) est généralement une composition aqueuse. Par composition aqueuse, on entend au sens de l'invention une composition comprenant plus de 20 % en poids d'eau, de préférence plus de 30 % en poids d'eau, et de manière encore plus avantageuse plus de 40 % en poids d'eau.

La composition oxydante (B) est généralement une composition aqueuse. La composition oxydante (B) comprend usuellement de l'eau qui représentent généralement de 10 à 98 % en poids, de préférence de 20 à 96 % en poids, de préférence de 50 à 95 % en poids, par rapport au poids total de la composition.

***pH du milieu***

Le pH de la composition (A) mise en œuvre dans le procédé selon l'invention varie généralement de 1 à 12. De façon préférée, le pH de la composition (A) selon l'invention est basique.
Par « pH basique » au sens de la présente invention, on entend un pH supérieur à 7.
De préférence, le pH de la composition (A) selon l'invention est supérieur à 8, et particulièrement va de 8,5 à 12. De préférence le pH de la composition est compris entre 9 et 12.
Habituellement, le pH de la composition (B), est inférieur à 7. Le pH de la composition (B) de l'invention est avantageusement compris entre 1 et 7, de préférence entre 1 et 4, et plus préférentiellement de 1,5 à 3,5.

*Ajusteur de pH*

Le milieu cosmétiquement acceptable peut être ajusté à la valeur désirée au moyen d'agents acidifiants ou alcalinisants habituellement utilisés en teinture des fibres kératiniques ou bien encore à l'aide de systèmes tampons classiques.
Parmi les agents acidifiants, on peut citer, à titre d'exemple, les acides minéraux comme par exemple l'acide chlorhydrique, (ortho)phosphorique, l'acide borique, l'acide nitrique, l'acide sulfurique ou les acides organiques comme par exemple les composés comprenant au moins une fonction acide sulfonique, une fonction acide phosphonique ou une fonction acide phosphorique, ou les composés à fonction acide carboxylique tels que ceux décrits précédemment.

***Autres additifs***

Les compositions mises en œuvre dans le procédé selon l'invention peut également renfermer divers additifs utilisés classiquement dans les compositions pour la coloration des cheveux, tels que des agents épaississants minéraux, et en particulier des charges telles que des argiles, le talc ; des agens épaississants organiques différents des gommes de scléroglucane ;des agents antioxydants ; des agents de pénétration ; des agents séquestrants ; des parfums ; des agents dispersants ; des agents filmogènes ; des céramides ; des agents conservateurs ; des agents opacifiants, des corps gras et/ou des colorants directs additionnels.
Les additifs ci-dessus sont en général présents en quantité comprise pour chacun d'eux entre 0,01 et 40 % en poids par rapport au poids de la composition, de préférence entre 0,1 et 20 % en poids par rapport au poids de la composition (A) et/ou de la composition (B).
Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

**Procédé de coloration**

Selon une variante préférée du procédé de l'invention, on applique sur les matières kératiniques, sèches ou humides, une composition prête à l'emploi obtenue par mélange extemporané, au moment de l'emploi, de la composition colorante (A) et de la composition oxydante (B) telles que définies précédemment le rapport pondéral R des quantités de (A) / (B) variant de 0,1 à 10 de préférence de 0,2 à 2, mieux de 0,3 à 1.
En outre, l'application de la composition prête à l'emploi sur les matières kératiniques (résultant du mélange extemporané des compositions colorantes (A) et oxydantes (B)) est laissé en place pour une durée, en général, de l'ordre de 1 minute à 1 heure, de préférence de 5 minutes à 30 minutes.
La température durant le procédé est classiquement comprise entre la température ambiante (entre 15 à 25°C) et 80°C, de préférence entre la température ambiante et 60°C.
A l'issue du traitement, les matières kératiniques sont éventuellement rincées à l'eau, subissent éventuellement un lavage suivi d'un rinçage à l'eau, avant d'être séchées ou laissées à sécher.
De préférences les fibres kératiniques sont des fibres kératiniques humaines, de préférence des cheveux humains.
Selon un mode particulier de l'invention, le ou les agents oxydants chimiques représente(nt) de préférence une teneur totale variant de 0,1 à 20 % en poids, de préférence de 0,5 à 15% en poids, ou encore plus préférentiellement de 1 à 10% en poids, par rapport au poids total de la composition prête à l'emploi.
Enfin, l'invention concerne un dispositif à plusieurs compartiments comprenant dans un premier compartiment

une composition colorante (A) comme décrite auparavant, et dans un second, une composition oxydante (B telle que décrite, auparavant.

En particulier, l'invention a également pour objet un dispositif (ou " kit ") à plusieurs compartiments permettant la mise en œuvre du procédéde coloration des fibres kératiniques, de préférence comprenant au moins deux compartiments, un premier compartiment contenant la composition colorante (A) telle que définie précédemment, et le second compartiment la composition oxydante (B) telle que définie précédemment, les compositions des compartiments étant destinées à être mélangées juste avant (extemporanément) l'emploi (c'est-à-dire juste avant application), pour donner la formulation après mélange prête à l'emploi ; en particulier le kit peut être un dispositif aérosol.

Les exemples suivants servent à illustrer l'invention sans toutefois présenter un caractère limitatif.

En particulier, la coloration des fibres kératiniques obtenue dans ces exemples peut être avantageusement évaluée dans le système CIE L* a* b*, au moyen d'un Spectrocolorimètre Datacolor Spectraflash SF600X.

Dans ce système L* a* b*, les trois paramètres désignent respectivement l'intensité de la couleur (L*), a* indique l'axe de couleur vert/rouge et b* l'axe de couleur bleu/jaune. Plus la valeur de L* est élevée, plus la couleur est claire. Plus la valeur de a* est élevée, plus la couleur est rouge, plus la valeur de b* est élevée, plus la couleur est jaune.

La variation (ou l'importance) de la coloration entre les mèches de cheveux non traitées et les mèches de cheveux après traitement est définie par le paramètre DE* et est calculé selon l'équation suivante :

$$DE^* = \sqrt{(L^* - L_o{}^*)^2 + (a^* - a_o{}^*)^2 + (b^* - b_o{}^*)^2} \quad \text{(i)}$$

Dans cette équation, les paramètres L*, a* et b* représentent les valeurs mesurées sur les mèches de cheveux après la coloration et les paramètres $L_0$*, $a_0$* et $b_0$* représentent les valeurs mesurées sur les mèches de cheveux non traitées. Plus la valeur de DE* est grande, meilleure est la coloration des fibres kératiniques.

Dans le système CIE L*, a*, b*, la chromaticité est calculée selon l'équation suivante:

$$C^* = \sqrt{a^{*2} + b^{*2}}$$

Plus la valeur de C* est élevée et plus la coloration est chromatique.

**Exemple 1 :**

**Composition colorante**

On a préparé les compositions colorantes suivantes à partir des ingrédients suivants dans les proportions suivantes indiquées en gramme de matière active :

| | Composition comparative C1 hors invention | Composition comparative C2 hors invention | Composition comparative C3 hors invention | Composition A1 (selon l'invention) |
|---|---|---|---|---|
| Ammonium Hydroxide | 2,47 | 2,47 | 2,47 | 2,47 |
| Ethanolamine | 4 | 4 | 4 | 4 |
| EDTA | 0,2 | 0,2 | 0,2 | 0,2 |
| Sodium Sulfite | 0,5 | 0,5 | 0,5 | 0,5 |
| Colorants d'oxydation | 1,401 | 1,401 | 1,401 | 1,401 |
| Fragance | qs | qs | qs | qs |
| Hexadimethrine Chloride | 0,3 | 0,3 | 0,3 | 0,3 |
| Polyquaternium -6 | 0,4 | 0,4 | 0,4 | 0,4 |

(suite)

| | Composition comparative C1 hors invention | Composition comparative C2 hors invention | Composition comparative C3 hors invention | Composition A1 (selon l'invention) |
|---|---|---|---|---|
| Cetyl hydroxyéthylcel lulose | 0,2 | 0,2 | 0,2 | 0,2 |
| Xanthan Gum | 1 | - | - | - |
| Algin | - | - | 1 | - |
| Sclerotium Gum | - | - | - | 1 |
| Hydroxypropyl cellulose | - | 1 | - | - |
| Eau | Qs 100 | Qs 100 | Qs 100 | Qs 100 |
| Glycérine | 10 | 10 | 10 | 10 |
| Cetrimonium Chloride | 0,25 | 0,25 | 0,25 | 0,25 |
| Caprylylcapryl Glucoside | 0,6 | 0,6 | 0,6 | 0,6 |
| Acide Ascorbique | 0,4 | 0,4 | 0,4 | 0,4 |

**Evaluation visuelle de la stabilité des compositions**

La stabilité des compositions colorantes a été évaluée en observant les compositions à T0 (immédiatement après préparation de la composition) puis après 2 mois de stockage à 45°C.

| | Composition C1 | Composition C2 | Composition C3 | Composition A1 |
|---|---|---|---|---|
| Observation à T0 à température ambiante (25°C) | Texture liquide Non homogène (déphasage) | Texture liquide Non homogène (déphasage) | Texture liquide Non homogène (déphasage) | Gel translucide Homogène (pas de déphasage) |
| Observation après 2 mois à 45°C | Texture liquide Non homogène (déphasage) | Texture liquide Non homogène (déphasage) | Texture liquide Non homogène (déphasage) | Gel translucide Homogène (pas de déphasage) |

On observe que la composition A1 selon l'invention est homogène et forme un gel translucide à T0. Après 2 mois à 45° la composition A1 selon l'invention est stable ; elle est homogène et translucide. Les compositions comparatives C1, C2 et C3 dans lesquelles la gomme de scléroglucane a été remplacée poids pour poids par un autre agent épaississant de type polysaccharide ne sont pas stables. En effet, elles ne sont pas homogènes, dès T0 on observe un déphasage de ces compositions.

**Exemple 2** : **compositions oxydantes**

*Préparation des compositions*

Les compositions C et B sont préparées avec les ingrédients et les teneurs tels qu'indiqués dans le ci-dessous.

La composition C est comparative et comprend de la gomme de xanthane. La composition B est selon l'invention et comprend de la gomme de scléroglucane.

Les teneurs sont indiquées en grammes de matière active sauf mention contraire.

| Ingrédients | Composition C (comparative.) | Composition B1 (invention.) |
|---|---|---|
| Gomme de xanthane | 1,5 | - |
| Gomme de scléroglucane | - | 1,5 |
| Etidronate de tétrasodium | 0,06 | 0,06 |
| Pyrophosphate de tétrasodium | 0,04 | 0,04 |
| Salicylate de sodium | 0,035 | 0,035 |
| Peroxyde d'hydrogène | 4,5 | 4,5 |
| Acide phosphorique | qs pH 2,2 ± 0,2 | qs pH 2,2 ± 0,2 |
| Eau | qsp 100 | qsp 100 |

Le T0 pour les caractérisations des compositions correspond à l'état du système 24 heures après l'ajustement du pH.

*Caractérisations des compositions*

La viscosité des compositions Blet C ont été mesurée au cours du stockage à pression atmosphérique, à 25°C et à 45°C, à T0 (après ajustement du pH), à un jour (T1), une semaine (T2) et deux semaines (T3).

On constate que la composition B1 selon l'invention présente une faible variation de la viscosité au cours du temps, contrairement à la composition comparative C dont la viscosité varie (diminue) de façon plus importante au cours du temps, la composition C est de plus en plus fluide ; aussi bien à température ambiante qu'à 45°C.

La composition B1 selon l'invention présente donc une stabilité plus importante que la composition comparative C qui comprend de la gomme de xanthane. La composition B1 forme d'un gel homogène et translucide (pas de déphasage) après deux mois à température ambiante qu'à 45°C.

**Exemple 3 :**

*Préparation des compositions oxydantes*

La composition B2 a été préparé avec les ingrédients et les teneurs tels qu'indiqués dans le tableau ci-dessous.

Les teneurs sont indiquées en grammes de matière active sauf mention contraire.

| Ingrédients | Composition B2 selon l'invention |
|---|---|
| Gomme de scléroglucane | 1,5 |
| Peroxyde d'hydrogène | 12 |
| Etidronate de tétrasodium | 0,06 |
| Acide phosphorique | qs pH 2,0 ± 0,2 |
| Eau désionisée | qsp 100 |

La composition B2 forme un gel homogène et translucide. Elle reste stable après 2 mois à 25°C et après 2 mois à 45°C. on n'observe pas de formation de bulles.

De plus, la viscosité de la composition B2 a été mesurée au cours du stockage à 45°C, à T0 (après ajustement du pH), à un jour (T1), une semaine (T2) et deux semaines (T3).

On constate que la composition B2 selon l'invention présente une faible variation de la viscosité au cours du temps.

**Exemple 4**

On a préparé la composition colorante suivante à partir des ingrédients suivants dans les proportions suivantes indiquées en gramme :

| | Composition A2 selon l'invention |
|---|---|
| ETHANOLAMINE | 3,23 |
| EDTA | 0,2 |
| SODIUM SULFITE | 0,5 |
| TOLUENE-2,5-DIAMINE | 1,03 |
| 2,4-DIAMINOPHENOXYETHANOL HCl | 0,021 |
| 2-METHYLRESORCINOL | 0,27 |
| N,N-BIS(2-HYDROXYETHYL)-p-PHENYLENEDIAMINE SULFATE | 0,13 |
| RESORCINOL | 0,28 |
| m-AMINOPHENOL | 0,45 |
| FRAGRANCE | qs |
| CETYL HYDROXYETHYLCELLULOSE | 0,2 |
| SCLEROTIUM GUM | 1 |
| EAU | qs 100 |
| GLYCERIN | 10 |
| COCO-BETAINE | 0,15 |
| CAPRYLYL/CAPRYL GLUCOSIDE | 0,6 |
| ASCORBIC ACID | 0,4 |

**Evaluation visuelle de la stabilité des compositions**

La stabilité des compositions colorantes a été évaluée en observant les compositions à T0 (immédiatement après préparation de la composition) puis après 2 mois de stockage à température ambiante (25°C), et après 2 mois de stockage à 45°C.

| | Composition A2 selon l'invention |
|---|---|
| Observation à T0 (immédiatement après préparation) | Homogène (Pas de Déphasage) Texture : Gel lisse |
| Observation après 2 mois à 25°C | Homogène (Pas de Déphasage) Texture : Gel lisse |
| Observation après 2 mois à 45°C | Homogène (Pas de Déphasage) Texture : Gel lisse |

On observe que la composition A2 du procédé selon l'invention sont homogènes et forment un gel translucide à T0. Après 2 mois à température ambiante ou à 45° C.

On a préparé la composition oxydante B3 suivantes à partir des ingrédients suivants dans les proportions suivantes indiquées en gramme.

| | Composition oxydante B3 selon l'invention |
|---|---|
| HYDROGEN PEROXIDE | 6 |
| PHOSPHORIC ACID | Qs pH 2,2 ± 0,2 |
| TETRASODIUM ETIDRONATE | 0,2 |
| TETRASODIUM PYROPHOSPHATE | 0,04 |
| SODIUM SALICYLATE | 0,035 |
| SCLEROTIUM GUM | 1,8 |

(suite)

| | Composition oxydante B3 selon l'invention |
|---|---|
| EAU | Qs 100 |

On observe que la composition B3 du procédé selon l'invention est homogène et forme un gel translucide à T0, après 2 mois à température ambiante ou à 45° C.

La composition A2 a été mélangée respectivement avec 1 fois son poids de composition oxydante B3

On observe un mélange facile des compositions et l'obtention d'un mélange homogène et translucide. Les mélanges ainsi obtenus ont été appliqués sur des mèches de cheveux naturels à 90% blancs.

Le rapport de bain « mélange/mèche » est respectivement de 10/1 (g/g).

Le mélange se répartit aisément de façon homogène sur les cheveux.

Les qualités d'usage sont bonnes : bon caractère mouillant/glissant, bonne facilité d'application, bonne adhérence racine, bonne consistance sur la tête, bonne facilité d'allongement le long des mèches de fibres.

Le temps de pause de 30 minutes, sur plaque chauffante réglée à 27°C. A l'issue du temps de pause, les mèches sont rincées puis séchées sous casque à 40°C.

Les cheveux sont ensuite facilement rincés, puis lavés avec un shampooing standard et séchés.

La couleur des mèches a été évaluée dans le système CIE L*a*b* au moyen d'un spectrocolorimètre Datacolor Spectraflash SF600X.

| | L* |
|---|---|
| Mélange composition A2 et B3 | 21,48 |

On obtient une coloration intense des fibres kératiniques.

## Revendications

1. Procédé de coloration des fibres kératiniques, en particulier des fibres kératiniques humaines telles que les cheveux mettant en œuvre l'application sur les fibres

   a) d'une composition colorante (A) comprenant :

      - un ou plusieurs colorant(s) d'oxydation ;
      - un ou plusieurs gomme(s) de scléroglucane, en une teneur totale en poids supérieure ou égale à 0,5% par rapport au poids total de la composition (A) ;
      - un ou plusieurs agent(s) alcalin(s) ; et

   b) d'une composition oxydante (B) comprenant :

      - un ou plusieurs agent(s) oxydant(s) chimique(s) et
      - une ou plusieurs gomme(s) de scléroglucane,;

   la composition oxydante (B) étant mélangée avec la composition colorante (A) juste avant (extemporanément) l'emploi.

2. Procédé selon la revendication 1, **caractérisé en ce que** la ou les gommes de scléroglucane représentent de 0,5 à 10 % en poids, plus préférentiellement de 0,5 à 5 % en poids, et encore plus préférentiellement de 0,5 à 3 % en poids, mieux de 0.5% à 2% en poids et encore plus préférentiellement de 0,7 à 1,5 % en poids, par rapport au poids total de la composition (A).

3. Procédé selon l'une quelconque des revendications précédentes, **caractérisé en ce que** le ou les colorants d'oxydation sont choisi(s) parmi les bases d'oxydation benzéniques de préférence choisies parmi les paraphénylènediamines, les bis-phénylalkylènediamines, les para-aminophénols, les ortho-aminophénols, les bases hétérocycliques et leurs sels d'addition ; éventuellement combinées à un ou plusieurs coupleurs de préférence choisis parmi les méta-phénylènediamines, les méta-aminophénols, les méta-diphénols, les coupleurs naphthaléniques, et les coupleurs hétérocycliques ainsi que leurs sels d'addition.

4. Procédé selon l'une quelconque des revendications précédentes, **caractérisé en ce que** le ou lesdits agents alcalins sont présents en une teneur totale allant de 1 à 20% en poids, plus préférentiellement de 3 à 18% en poids, mieux de 5 à 16 % en poids par rapport au poids total de la composition colorante (A).

5. Procédé selon l'une quelconque des revendications précédentes, **caractérisé en ce que** le ou les agents alcalins sont choisis parmi les agents alcalins minéraux et/ou organiques, de préférence parmi un mélange d'au moins un agent alcalin minéral et d'au moins un agent alcalin organique ;

6. Procédé selon l'une quelconque des revendications précédentes, **caractérisé en ce que** le ou les agents alcalins minéraux sont choisis parmi l'ammoniaque (ou agents précurseurs d'ammoniac comme les sels d'ammonium), les silicates, les phosphates, les carbonates ou bicarbonates de métaux alcalins ou alcalino-terreux, tels que métasilicates de métaux alcalins ou alcalino terreux, les carbonates ou bicarbonates de sodium ou de potassium, les hydroxydes de sodium ou de potassium, ou leurs mélanges, de préférence parmi l'ammoniaque et/ou les métasilicates de métaux alcalins ou alcalino terreux.

7. Procédé selon l'une quelconque des revendications précédentes, **caractérisée** en que le ou les agents alcalins est/sont choisis parmi l'ammoniaque, les métasilicates de métaux alcalins ou alcalino terreux les alcanolamines, les acides aminés, et leurs mélanges, de préférence la composition comprend de l'ammoniaque (hydroxyde d'ammonium) et une ou plusieurs alcanolamine(s), de préférence la monoethanolamine.

8. Procédé selon l'une quelconque des revendications précédentes, **caractérisé en ce que** la composition colorante (A) comprend un ou plusieurs agents tensioactifs, de préférence choisis parmi les tensioactifs cationiques et/ou non ioniques, le ou lesdits tensioactifs est/sont de préférence présents en une teneur totale allant de 0,01 à 20% , plus préférentiellement de 0,05 à 10% en poids, mieux de 0,1 à 5% en poids par rapport au poids totale de la composition colorante (A).

9. Procédé selon l'une quelconque des revendications 8, **caractérisé en ce que** le ou ledits tensioactifs est/sont non ioniques(s) et sont de préférence choisis parmi :

- les alcools gras oxyéthylénés, comportant au moins une chaîne alkyle en C8 à C40, notamment en C8-C20, encore mieux en C10-C18 , saturée ou non, linéaire ou ramifiée, et comprenant de 1 à 100 moles d'oxyde d'éthylène, de préférence de 2 à 50, plus particulièrement de 2 à 40 moles, voire de 3 à 20 moles d'oxyde d'éthylène;, et
- les alkylpolyglycosides, de préférence les (alkyl C6-C24)(poly)glycosides, et plus particulièrement les (alkyl C8-C18)(poly)glycosides ;
- et leurs mélanges ;

et plus préférentiellement encore parmi les alkylpolyglycosides, de préférence parmi les (alkyl C6-C24)(poly)glycosides, tels que les (alkyl C8-C18)(poly)glycoside.

10. Procédé selon l'une quelconque des revendications précédentes, **caractérisé en ce que** la composition colorante (A) comprend un ou plusieurs polymère(s) associatif(s), de préférence non ioniques(s) ; de préférence le ou ledits polymères associatifs étant présent(s) en une teneur totale allant de 0,01 et 10%, et encore plus préférentiellement entre 0,05 et 5% du poids total de la composition, mieux entre 0,1 et 2% en poids par rapport au poids total de la composition de la composition colorante (A).

11. Procédé selon la revendication précédente, **caractérisé en ce que** ledit ou lesdits polymères associatifs non ioniques sont choisis parmi les celluloses modifiées par des groupements comportant au moins une chaîne grasse, de préférence choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et leurs mélanges, de préférence la cétylhydroxyethylcellulose.

12. Procédé selon l'une quelconque des revendications précédentes **caractérisé en ce que** la composition colorante (A) comprend un ou plusieurs polymère(s) cationique(s) choisi(s) parmi :

- (1) les homopolymères de dialkyl diallyl ammonium, de préférence les homopolymères de

diméthyldiallylammonium, ; et/ou

- (2) les polymères cationiques qui sont constitués de motifs récurrents répondant à la formule :

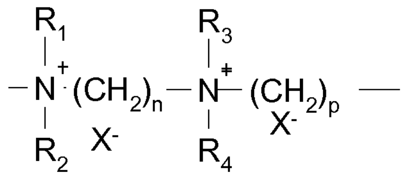 (IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique ; de préférence le polmyère composé de motifs formule (IV) pour lequel R1, R2, R3 et R4 représentent un radical méthyle, n=3, p=6 et X = Cl, dénommé Hexadimethrine chloride .

**13.** Procédé selon l'une quelconque des revendications précédentes, **caractérisé en ce que** la ou les gommes de scléroglucane représentent de 0,5 à 10 % en poids, plus préférentiellement de 0,5 à 5 % en poids, et encore plus préférentiellement de 0,5 à 3 % en poids, encore plus préférentiellement, de 0,7 à 2% en poids, par rapport au poids total de la composition oxydante (B)

**14.** Procédé selon la revendication précédente **caractérisé en ce que** le ou les agents oxydants chimique sont choisis parmi le peroxyde d'hydrogène et/ou un ou plusieurs système(s) générateur(s) de peroxyde d'hydrogène, de préférence le peroxyde d'hydrogène.

**15.** Dispositif (ou " kit ") à plusieurs compartiments comprenant au moins deux compartiments, un premier compartiment contenant la composition colorante (A) et un second compartiment contenant la composition oxydante (B), les compositions (A) et (B) étant telles que définis à l'une quelconque des revendications 1 à 14.

**16.** Kit selon la revendication 15 sous forme d'un dispositif aérosol.

**Patentansprüche**

**1.** Verfahren zum Färben von Keratinfasern, insbesondere menschlichen Keratinfasern wie dem Haar, bei dem man auf die Fasern

a) eine Färbezusammensetzung (A), umfassend:

- einen oder mehrere Oxidationsfarbstoffe;
- ein oder mehrere Skleroglucan-Gummen in einem Gesamtgewichtsgehalt größer oder gleich 0,5 %, bezogen auf das Gesamtgewicht der Zusammensetzung (A);
- ein oder mehrere alkalische Mittel; und

b) eine oxidierende Zusammensetzung (B) umfassend:

- ein oder mehrere chemische Oxidationsmittel und
- ein oder mehrere Skleroglucan-Gummen;

aufbringt, wobei die oxidierende Zusammensetzung (B) kurz vor der Anwendung (extemporal) mit der Färbezusammensetzung (A) gemischt wird.

**2.** Verfahren nach Anspruch 1, **dadurch gekennzeichnet, dass** das Skleroglucan-Gummi bzw. die Skleroglucan-Gummen 0,5 bis 10 Gew.-%, weiter bevorzugt 0,5 bis 5 Gew.-% und noch weiter bevorzugt 0,5 bis 3 Gew.-%, noch besser 0,5 bis 2 Gew.-% und noch weiter bevorzugt 0,7 bis 1,5 Gew.-%, bezogen auf das Gesamtgewicht der Zusammensetzung (A), ausmacht bzw. ausmachen.

**3.** Verfahren nach einem der vorhergehenden Ansprüche, **dadurch gekennzeichnet, dass** der Oxidationsfarbstoff bzw. die Oxidationsfarbstoffe aus Benzol-Oxidationsbasen, vorzugsweise ausgewählt aus para-Phenylendiaminen,

Bisphenylalkylendiaminen, para-Aminophenolen, ortho-Aminophenolen, heterocyclischen Basen und Additionssalzen davon; gegebenenfalls in Kombination mit einem oder mehreren Kupplern, vorzugsweise ausgewählt aus meta-Phenylendiaminen, meta-Aminophenolen, meta-Diphenolen, naphthalin-Kupplern und heterocyclischen Kupplern sowie Additionssalzen davon; ausgewählt ist bzw. sind.

**4.** Verfahren nach einem der vorhergehenden Ansprüche, **dadurch gekennzeichnet, dass** das alkalische Mittel bzw. die alkalischen Mittel in einem Gesamtgehalt im Bereich von 1 bis 20 Gew.-%, weiter bevorzugt von 3 bis 18 Gew.-%, noch besser von 5 bis 16 Gew.-%, bezogen auf das Gesamtgewicht der Färbezusammensetzung (A), vorliegt bzw. vorliegen.

**5.** Verfahren nach einem der vorhergehenden Ansprüche, **dadurch gekennzeichnet, dass** das alkalische Mittel bzw. die alkalischen Mittel aus mineralischen und/oder organischen alkalischen Mitteln, vorzugsweise aus einer Mischung von mindestens einem mineralischen alkalischen Mittel und mindestens einem organischen alkalischen Mittel, ausgewählt ist bzw. sind.

**6.** Verfahren nach einem der vorhergehenden Ansprüche, **dadurch gekennzeichnet, dass** das mineralische alkalische Mittel bzw. die mineralischen alkalischen Mittel aus wässrigem Ammoniak (oder Ammoniak-Vorstufen wie Ammoniumsalzen), Alkalimetall- oder Erdalkalimetallsilikaten, -phosphaten, -carbonaten oder -hydrogencarbonaten, wie Alkali- oder Erdalkalimetallmetasilikaten, Natrium- oder Kaliumcarbonat oder -hydrogencarbonat, Natrium- oder Kaliumhydroxid oder Mischungen davon, vorzugsweise aus wässrigem Ammoniak und/oder Alkali- oder Erdalkalimetallmetasilikaten, ausgewählt ist bzw. sind.

**7.** Verfahren nach einem der vorhergehenden Ansprüche, **dadurch gekennzeichnet, dass** das alkalische Mittel bzw. die alkalischen Mittel aus wässrigem Ammoniak, Alkali- oder Erdalkalimetallmetasilikaten, Alkanolaminen, Aminosäuren und Mischungen davon ausgewählt ist bzw. sind und die Zusammensetzung vorzugsweise wässriges Ammoniak (Ammoniumhydroxid) und ein oder mehrere Alkanolamine, vorzugsweise Monoethanolamin, umfasst.

**8.** Verfahren nach einem der vorhergehenden Ansprüche, **dadurch gekennzeichnet, dass** die Färbezusammensetzung (A) ein oder mehrere Tenside umfasst, vorzugsweise ausgewählt aus kationischen und/oder nichtionischen Tensiden, wobei das Tensid bzw. die Tenside vorzugsweise in einem Gesamtgehalt im Bereich von 0,01 bis 20 Gew.-%, weiter bevorzugt von 0,05 bis 10 Gew.-%, noch besser von 0,1 bis 5 Gew.-%, bezogen auf das Gesamtgewicht der Färbezusammensetzung (A), vorliegen.

**9.** Verfahren nach einem der Ansprüche 8, **dadurch gekennzeichnet, dass** das Tensid bzw. die Tenside nichtionisch ist bzw. sind und vorzugsweise aus

- oxyethyleniertgen Fettalkoholen mit mindestens einer gesättigten oder ungesättigten, linearen oder verzweigten C8- bis zu C40-, insbesondere C8-C20-, noch besser C10-C18-Alkylkette und mit 1 bis 100 mol Ethylenoxid, vorzugsweise 2 bis 50, spezieller 2 bis 40 mol oder sogar 3 bis 20 mol Ethylenoxid und
- Alkylpolyglycosiden, vorzugsweise (C6-C24-Alkyl)(poly)glycosiden und spezieller (C8-C18-Alkyl)(poly)glycosiden,
- und Mischungen davon

und noch weiter bevorzugt aus Alkylpolyglycosiden, vorzugsweise aus (C6-C24-Alkyl) (poly)glycosiden, wie (C8-C18-Alkyl)(poly)glycosiden,
ausgewählt ist bzw. sind.

**10.** Verfahren nach einem der vorhergehenden Ansprüche, **dadurch gekennzeichnet, dass** die Färbezusammensetzung (A) ein oder mehrere vorzugsweise nichtionische assoziative Polymere umfasst; wobei das assoziative Polymer bzw. die assoziativen Polymere in einem Gesamtgehalt im Bereich von 0,01 und 10 % und noch weiter bevorzugt zwischen 0,05 und 5 % des Gesamtgewichts der Zusammensetzung, noch besser zwischen 0,1 und 2 Gew.-%, bezogen auf das Gesamtgewicht der Zusammensetzung der Färbezusammensetzung (A), vorliegt bzw. vorliegen.

**11.** Verfahren nach dem vorhergehenden Anspruch, **dadurch gekennzeichnet, dass** das nichtionische assoziative Polymer bzw. die nichtionischen assoziativen Polymere aus Cellulosen, die durch Gruppen mit mindestens einer Fettkette modifiziert sind, vorzugsweise aus Hydroxyethylcellulosen, die durch Gruppen mit mindestens einer Fettkette wie Alkyl-, Arylalkyl-, Alkylarylgruppen oder Mischungen davon modifiziert sind, wobei es sich bei den Alkylgruppen vorzugsweise um $C_8$-$C_{22}$-Alkylgruppen handelt, und Mischungen davon, vorzugsweise Cetylhydroxyethylcellulose, ausgewählt ist bzw. sind.

**12.** Verfahren nach einem der vorhergehenden Ansprüche, **dadurch gekennzeichnet, dass** die Färbezusammensetzung (A) ein oder mehrere kationische Polymere umfasst, die ausgewählt ist bzw. sind aus:

- (1) Dialkyldiallylammonium-Homopolymeren, vorzugsweise Dimethyldiallylammonium-Homopolymeren, und/oder
- (2) kationischen Polymeren, die aus Wiederholungseinheiten aufgebaut sind, die der Formel:

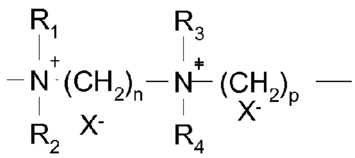 (IV)

entsprechen, wobei R1, R2, R3 und R4 gleich oder verschieden sind und für einen Alkyl- oder Hydroxyalkylrest mit 1 bis 4 Kohlenstoffatomen stehen, n und p ganze Zahlen im Bereich von 2 bis 20 sind und X- ein Anion ist, das sich von einer Mineralsäure oder organischen Säure ableitet; vorzugsweise dem Polymer, das aus Einheiten der Formel (IV) aufgebaut ist, für die R1, R2, R3 und R4 für einen Methylrest stehen, n = 3, p = 6 und X = Cl, mit der Bezeichnung Hexadimethrinchlorid.

**13.** Verfahren nach einem der vorhergehenden Ansprüche, wobei das Skleroglucan-Gummi bzw. die Skleroglucan-Gummen 0,5 bis 10 Gew.-%, weiter bevorzugt 0,5 bis 5 Gew.-% und noch weiter bevorzugt 5 bis 3 Gew.-%, noch weiter bevorzugt 0,7 bis 2 Gew.-%, bezogen auf das Gesamtgewicht der oxidierenden Zusammensetzung (B), ausmacht bzw. ausmachen.

**14.** Verfahren nach dem vorhergehenden Anspruch, **dadurch gekennzeichnet, dass** das chemische Oxidationsmittel bzw. die chemischen Oxidationsmittel aus Wasserstoffperoxid und/oder einem oder mehreren wasserstoffperoxiderzeugenden Systemen, vorzugsweise aus Wasserstoffperoxid, ausgewählt ist bzw. sind.

**15.** Vorrichtung (oder "Kit") mit mehreren Kompartimenten, umfassend mindestens zwei Kompartimente, wobei ein erstes Kompartiment die Färbezusammensetzung (A) enthält und ein zweites Kompartiment die oxidierende Zusammensetzung (B) enthält, wobei die Zusammensetzungen (A) und (B) wie in einem der Ansprüche 1 bis 14 definiert sind.

**16.** Kit nach Anspruch 15 in Form einer Aerosolvorrichtung.

## Claims

**1.** Process for dyeing keratin fibers, in particular human keratin fibers such as the hair, involving the application to the fibers

a) of a dye composition (A) comprising:

- one or more oxidation dyes;
- one or more scleroglucan gums in a total weight content of greater than or equal to 0.5% relative to the total weight of composition (A);
- one or more alkaline agent(s); and

b) of an oxidizing composition (B) comprising:

- one or more chemical oxidizing agents; and
- one or more scleroglucan gums;

the oxidizing composition (B) being mixed with the dye composition (A) just before use (extemporaneously).

**2.** Process according to Claim 1, **characterized in that** the scleroglucan gum(s) represent from 0.5% to 10% by weight,

more preferentially from 0.5% to 5% by weight, even more preferentially from 0.5% to 3% by weight, better still from 0.5% to 2% by weight and even more preferentially from 0.7% to 1.5% by weight, relative to the total weight of composition (A).

**3.** Process according to either of the preceding claims, **characterized in that** the oxidation dye(s) are chosen from benzene-based oxidation bases, preferably chosen from para-phenylenediamines, bis(phenyl)alkylenediamines, para-aminophenols, ortho-aminophenols, heterocyclic bases and the addition salts thereof; optionally combined with one or more couplers preferably chosen from meta-phenylenediamines, meta-aminophenols, meta-diphenols, naphthalene-based couplers, heterocyclic couplers, and also the addition salts thereof.

**4.** Process according to any one of the preceding claims, **characterized in that** said alkaline agent(s) are present in a total content ranging from 1% to 20% by weight, more preferentially from 3% to 18% by weight and better still from 5% to 16% by weight, relative to the total weight of the dye composition (A).

**5.** Process according to any one of the preceding claims, **characterized in that** the alkaline agent(s) are chosen from mineral and/or organic alkaline agents, preferably from a mixture of at least one mineral alkaline agent and of at least one organic alkaline agent.

**6.** Process according to any one of the preceding claims, **characterized in that** the mineral alkaline agent (s) are chosen from aqueous ammonia (or ammonia precursors such as ammonium salts), alkali metal or alkaline-earth metal silicates, phosphates, carbonates or bicarbonates, such as alkali metal or alkaline-earth metal metasilicates, sodium or potassium carbonate or bicarbonate, sodium or potassium hydroxide, or mixtures thereof, preferably from aqueous ammonia and/or alkali metal or alkaline-earth metal metasilicates.

**7.** Process according to any one of the preceding claims, **characterized in that** the alkaline agent(s) is/are chosen from aqueous ammonia, alkali metal or alkaline-earth metal metasilicates, alkanolamines, amino acids, and mixtures thereof; preferably, the composition comprises aqueous ammonia (ammonium hydroxide) and one or more alkanolamines, preferably monoethanolamine.

**8.** Process according to any one of the preceding claims, **characterized in that** the dye composition (A) comprises one or more surfactants, preferably chosen from cationic and/or nonionic surfactants; said surfactant(s) is/are preferably present in a total content ranging from 0.01% to 20%, more preferentially from 0.05% to 10% by weight and better still from 0.1% to 5% by weight relative to the total weight of the dye composition (A).

**9.** Process according to any one of Claims 8, **characterized in that** the surfactant(s) is/are nonionic and are preferably chosen from:

- saturated or unsaturated, linear or branched, oxyethylenated fatty alcohols including at least one C8 to C40, notably C8-C20 and better still C10-C18 alkyl chain, and comprising from 1 to 100 mol of ethylene oxide, preferably from 2 to 50, more particularly from 2 to 40 mol, or even from 3 to 20 mol of ethylene oxide; and
- alkylpolyglycosides, preferably (C6-C24 alkyl)(poly)glycosides, and more particularly (C8-C18 alkyl)(poly)glycosides;
- and mixtures thereof;

and even more preferentially from alkylpolyglycosides, preferably from (C6-C24 alkyl)(poly)glycosides such as (C8-C18 alkyl)(poly)glycosides.

**10.** Process according to any one of the preceding claims, **characterized in that** the dye composition (A) comprises one or more associative polymers, which are preferably nonionic; preferably, said associative polymer(s) are present in a total content ranging from 0.01% and 10% by weight, even more preferentially between 0.05% and 5% of the total weight of the composition, and better still between 0.1% and 2% by weight relative to the total weight of the composition of the dye composition (A).

**11.** Process according to the preceding claim, **characterized in that** said nonionic associative polymer(s) are chosen from celluloses modified with groups including at least one fatty chain, preferably chosen from hydroxyethylcelluloses modified with groups including at least one fatty chain such as alkyl, arylalkyl or alkylaryl groups or mixtures thereof, and in which the alkyl groups are preferably $C_8$-$C_{22}$, and mixtures thereof, preferably cetylhydroxyethylcellulose.

**12.** Process according to any one of the preceding claims, **characterized in that** the dye composition (A) comprises one or more cationic polymers chosen from:

- (1) dialkyldiallylammonium homopolymers, preferably dimethyldiallylammonium homopolymers; and/or
- (2) cationic polymers that are constituted of repeating units corresponding to the formula:

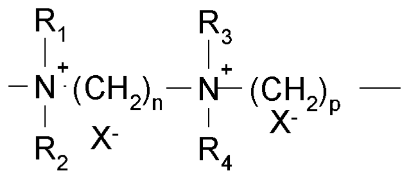

(IV)

in which R1, R2, R3 and R4, which may be identical or different, denote an alkyl or hydroxyalkyl radical containing from 1 to 4 carbon atoms, n and p are integers ranging from 2 to 20, and X- is an anion derived from a mineral or organic acid; preferably, the polymer composed of units of formula (IV) for which R1, R2, R3 and R4 represent a methyl radical, n = 3, p = 6 and X = Cl, known as hexadimethrine chloride.

**13.** Process according to any one of the preceding claims, **characterized in that** the scleroglucan gum(s) represent from 0.5% to 10% by weight, more preferentially from 0.5% to 5% by weight, even more preferentially from 0.5% to 3% by weight, and even more preferentially from 0.7% to 2% by weight, relative to the total weight of the oxidizing composition (B).

**14.** Process according to the preceding claim, **characterized in that** the chemical oxidizing agent (s) are chosen from hydrogen peroxide and/or one or more systems for generating hydrogen peroxide, preferably hydrogen peroxide.

**15.** Multi-compartment device (or "kit") comprising at least two compartments, a first compartment containing the dye composition (A) and a second compartment containing the oxidizing composition (B), compositions (A) and (B) being as defined in any one of Claims 1 to 14.

**16.** Kit according to Claim 15, in the form of an aerosol device.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 2010192969 A1, DEGEORGE MICHAEL
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A
- US 4874554 A
- US 4137180 A